# EUROPEAN PATENT APPLICATION

(11) **EP 3 783 349 A1**
(43) Date of publication of application: **24.02.2021**
(21) Application number: 19788620.3
(22) Date of filing: 15.04.2019
(51) Int. Cl.: G01N 27/00, C12M 1/00, C12N 15/09, C12Q 1/6869

(54) **FLUID CHIP AND ANALYSIS DEVICE**

(30) Priority: 19.04.2018 JP 2018081011
(71) Applicant: TEI Solutions Inc., Tsukuba-shi, Ibaraki 305-8569 (JP)
(72) Inventor: IKEDA, Shuji, Koganei-shi, Tokyo 184-0015 (JP); HASHIMOTO, Naotaka, Koganei-shi, Tokyo 184-0015 (JP)
(74) Representative: Bandpay & Greuter
(86) International application number: PCT/JP2019/016216
(87) International publication number: WO 2019/203201

(57) **Abstract**

Provided are a fluid chip provided in an analysis device analyzing a minute amount of sample and capable of reducing the size of the analysis device and an analysis device including the fluid chip. A fluid chip 10 includes an intra-substrate flow path provided in a substrate 21, a surface-side insulating film 22 as an insulating film provided on a surface of the substrate 21, an inflow opening portion 22a provided on an upstream side of the intra-substrate flow path and allowing a sample to flow into the intra-substrate flow path, and an outflow opening portion 22b provided on a downstream side of the intra-substrate flow path and allowing the sample to flow out of the intra-substrate flow path. The inflow opening portion 22a and the outflow opening portion 22b are provided in the surface-side insulating film 22 and interconnected via the intra-substrate flow path.

## Description

### Technical Field

The present invention relates to a fluid chip and an analysis device.

### Background Art

An analysis device including a fluid chip provided with a flow path having a through hole having a nanosize diameter (also referred to as a nanopore) is known as an analysis device analyzing a minute amount of sample. For example, the analysis device that is described in Patent Literature 1 analyzes the base sequence of deoxyribonucleic acid (DNA) or the like by using a silicon substrate where a nanopore having a diameter of several to tens of nanometers is provided in a flow path as a fluid chip. The flow path penetrates the silicon substrate. In addition, the inner wall of the flow path is inclined and the opening portion on the surface side of the silicon substrate is smaller than the opening portion on the back surface side of the silicon substrate.

In Patent Literature 1, the silicon substrate is provided between a supply portion where the DNA is supplied and a collection portion where the DNA is collected. The supply portion is connected to the flow path via the opening portion provided on the surface side of the silicon substrate. The collection portion is connected to the flow path via the opening portion provided on the back surface side of the silicon substrate. An electrode pair for performing DNA electrophoresis is provided in the supply portion and the collection portion. A voltage is applied to the electrode pair, the change in current value at a time when the DNA passes through the nanopore by electrophoresis is measured, and the base sequence of the DNA or the like is analyzed as a result.

### Citation List

### Patent Literature

Patent Literature 1: JP-A-2015-198652

### Summary of Invention

### Technical Problem

However, in Patent Literature 1, the electrode pair is provided so as to sandwich the silicon substrate from both surfaces, and thus it is necessary to separate holding members for individually holding the electrodes from each other and this necessity is a barrier to a reduction in the size of the analysis device.

An object of the invention is to provide a fluid chip provided in an analysis device analyzing a minute amount of sample and capable of reducing the size of the analysis device and an analysis device including the fluid chip.

### Solution to Problem

A fluid chip of the invention includes an intra-substrate flow path provided in a substrate, an insulating film provided on a surface of the substrate, an inflow opening portion provided on an upstream side of the intra-substrate flow path and allowing a sample to flow into the intra-substrate flow path, and an outflow opening portion provided on a downstream side of the intra-substrate flow path and allowing the sample to flow out of the intra-substrate flow path. The inflow opening portion and the outflow opening portion are provided in the insulating film and interconnected via the intra-substrate flow path.

Another fluid chip of the invention includes an intra-substrate flow path provided in a substrate, an insulating film provided on a surface of the substrate, and an inflow opening portion provided in the insulating film and allowing a sample to flow into the intra-substrate flow path. The intra-substrate flow path has a surface opening provided in the surface of the substrate, a back surface opening provided in a back surface of the substrate, a first inner wall provided between the surface opening and the back surface opening and inclined with respect to the back surface of the substrate, and a second inner wall provided downstream of the first inner wall between the surface opening and the back surface opening and perpendicular to the back surface of the substrate.

Another fluid chip of the invention includes an intra-substrate flow path provided in a substrate, an insulating film provided on a surface of the substrate, an inflow opening portion provided in the insulating film and allowing a sample to flow into the intra-substrate flow path, and a conductive film provided in contact with the insulating film. The conductive film has a conductive film opening portion connected to the inflow opening portion.

An analysis device of the invention includes the fluid chip, an upper-side sheet provided on a surface of the fluid chip, a supply portion where the sample is supplied, and a collection portion where the sample is collected. The upper-side sheet has a first upper-side flow path interconnecting the supply portion and the inflow opening portion and a second upper-side flow path interconnecting the collection portion and the outflow opening portion. A flow channel allowing the sample to flow between the supply portion and the collection portion is formed.

Another analysis device of the invention includes the fluid chip, an upper-side sheet provided on a surface of the fluid chip, a lower-side sheet provided on a back surface of the fluid chip, a supply portion where the sample is supplied, and a collection portion where the sample is collected. The upper-side sheet has a first upper-side flow path interconnecting the supply portion and the inflow opening portion and a second upper-side flow path interconnecting the collection portion and the outflow opening portion. The intra-substrate flow path has an upstream flow path connected to the inflow opening portion and a downstream flow path connected to the outflow opening portion. The lower-side sheet has a lower-side flow path interconnecting the upstream flow path and the downstream flow path. A flow channel allowing the sample to flow between the supply portion and the collection portion is formed.

Another analysis device of the invention includes a fluid chip having an intra-substrate flow path penetrating a substrate having a surface on which an insulating film is provided, an upper-side sheet provided on a surface of the fluid chip, a lower-side sheet provided on a back surface of the fluid chip, a chip frame provided between the upper-side sheet and the lower-side sheet and holding the fluid chip, a supply portion where a sample is supplied, and a collection portion where the sample is collected. The upper-side sheet has a first upper-side flow path connected to the supply portion and a second upper-side flow path connected to the collection portion. The chip frame has a connection hole connected to the second upper-side flow path. The insulating film has an inflow opening portion connected to the first upper-side flow path and allowing the sample to flow into the intra-substrate flow path. The lower-side sheet has a lower-side flow path interconnecting the intra-substrate flow path and the connection hole. A flow channel allowing the sample to flow between the supply portion and the collection portion is formed.

### Advantageous Effects of Invention

According to the invention, the inflow opening portion connected to the supply portion where the sample is supplied and the outflow opening portion connected to the collection portion where the sample is collected are provided in the same surface of the substrate. As a result, an electrode pair can be disposed on the same surface, and thus the analysis device can be reduced in size.

### Brief Description of Drawings

Fig. 1 is a schematic cross-sectional view illustrating an analysis device in which the invention is implemented.
Fig. 2 is a plan view of a fluid chip.
Fig. 3 is an explanatory diagram illustrating a surface pattern forming step.
Fig. 4 is an explanatory diagram illustrating a back surface pattern forming step.
Fig. 5 is an explanatory diagram illustrating an intra-substrate flow path forming step.
Fig. 6 is an exploded perspective view illustrating an analysis device in which a fluid chip of a second embodiment is implemented.
Fig. 7 is a schematic cross-sectional view illustrating the analysis device in which the fluid chip of the second embodiment is implemented.
Fig. 8 is a schematic cross-sectional view illustrating the analysis device in which another fluid chip of the second embodiment is implemented.
Fig. 9 is a schematic cross-sectional view illustrating a fluid chip of a third embodiment.
Fig. 10 is an explanatory diagram illustrating a preparation step of the third embodiment.
Fig. 11 is an explanatory diagram illustrating a surface pattern forming step of the third embodiment.
Fig. 12 is an explanatory diagram illustrating a back surface pattern forming step of the third embodiment.
Fig. 13 is an explanatory diagram illustrating an intra-substrate flow path forming step of the third embodiment.
Fig. 14 is a schematic cross-sectional view illustrating an analysis device in which a fluid chip of a fourth embodiment is implemented.
Fig. 15A is a plan view illustrating a first insulating film forming step for forming an inflow opening portion.
Fig. 15B is a cross-sectional view taken along line B-B of Fig. 15A.
Fig. 15C is a cross-sectional view taken along line C-C of Fig. 15A.
Fig. 16A is a plan view illustrating a second insulating film forming step for forming the inflow opening portion.
Fig. 16B is a cross-sectional view taken along line B-B of Fig. 16A.
Fig. 16C is a cross-sectional view taken along line C-C of Fig. 16A.
Fig. 17A is a plan view illustrating a third insulating film forming step for forming the inflow opening portion.
Fig. 17B is a cross-sectional view taken along line B-B of Fig. 17A.
Fig. 17C is a cross-sectional view taken along line C-C of Fig. 17A.
Fig. 18A is a plan view illustrating an insulating film processing step for forming the inflow opening portion.
Fig. 18B is a cross-sectional view taken along line B-B of Fig. 18A.
Fig. 18C is a cross-sectional view taken along line C-C of Fig. 18A.
Fig. 19A is a plan view illustrating an insulating film removing step for forming the inflow opening portion.
Fig. 19B is a cross-sectional view taken along line B-B of Fig. 19A.
Fig. 19C is a cross-sectional view taken along line C-C of Fig. 19A.
Fig. 20A is a plan view illustrating another inflow opening portion.
Fig. 20B is a cross-sectional view taken along line B-B of Fig. 20A.
Fig. 20C is a cross-sectional view taken along line C-C of Fig. 20A.
Fig. 21A is a plan view illustrating a first insulating film forming step for forming another inflow opening portion.
Fig. 21B is a cross-sectional view taken along line B-B of Fig. 21A.
Fig. 21C is a cross-sectional view taken along line C-C of Fig. 21A.
Fig. 22A is a plan view illustrating a second insulating film forming step for forming another inflow opening portion.
Fig. 22B is a cross-sectional view taken along line B-B of Fig. 22A.
Fig. 22C is a cross-sectional view taken along line C-C of Fig. 22A.
Fig. 23A is a plan view illustrating a third insulating film forming step for forming another inflow opening portion.
Fig. 23B is a cross-sectional view taken along line B-B of Fig. 23A.
Fig. 23C is a cross-sectional view taken along line C-C of Fig. 23A.
Fig. 24A is a plan view illustrating a groove forming step for forming another inflow opening portion.
Fig. 24B is a cross-sectional view taken along line B-B of Fig. 24A.
Fig. 24C is a cross-sectional view taken along line C-C of Fig. 24A.
Fig. 25A is a plan view illustrating a fourth insulating film forming step for forming another inflow opening portion.
Fig. 25B is a cross-sectional view taken along line B-B of Fig. 25A.
Fig. 25C is a cross-sectional view taken along line C-C of Fig. 25A.
Fig. 26A is a plan view illustrating a fifth insulating film forming step for forming another inflow opening portion.
Fig. 26B is a cross-sectional view taken along line B-B of Fig. 26A.
Fig. 26C is a cross-sectional view taken along line C-C of Fig. 26A.
Fig. 27A is a plan view illustrating an insulating film processing step for forming another inflow opening portion.
Fig. 27B is a cross-sectional view taken along line B-B of Fig. 27A.
Fig. 27C is a cross-sectional view taken along line C-C of Fig. 27A.
Fig. 28 is an exploded perspective view illustrating an analysis device of a fifth embodiment.
Fig. 29 is a schematic cross-sectional view illustrating an analysis device in which a fluid chip of a sixth embodiment is implemented.
Fig. 30 is an explanatory diagram illustrating a surface pattern forming step of the sixth embodiment.
Fig. 31 is an explanatory diagram illustrating a back surface pattern forming step of the sixth embodiment.
Fig. 32 is an explanatory diagram illustrating a first etching step of the sixth embodiment.
Fig. 33 is an explanatory diagram illustrating an inner wall protective film forming step of the sixth embodiment.
Fig. 34 is an explanatory diagram illustrating the inner wall protective film forming step of the sixth embodiment.
Fig. 35 is an explanatory diagram illustrating a second etching step of the sixth embodiment.
Fig. 36 is a schematic cross-sectional view illustrating a fluid chip of a seventh embodiment.
Fig. 37 is an explanatory diagram illustrating a surface pattern forming step of the seventh embodiment.
Fig. 38 is an explanatory diagram illustrating a back surface pattern forming step of the seventh embodiment.
Fig. 39 is an explanatory diagram illustrating a first etching step of the seventh embodiment.
Fig. 40 is an explanatory diagram illustrating an inner wall protective film forming step of the seventh embodiment.
Fig. 41 is an explanatory diagram illustrating a second etching step of the seventh embodiment.
Fig. 42 is a schematic cross-sectional view illustrating a fluid chip of an eighth embodiment.
Fig. 43 is an explanatory diagram illustrating a surface pattern forming step of the eighth embodiment.
Fig. 44 is an explanatory diagram illustrating a back surface pattern forming step of the eighth embodiment.
Fig. 45 is an explanatory diagram illustrating a first etching step of the eighth embodiment.
Fig. 46 is an explanatory diagram illustrating a second etching step of the eighth embodiment.
Fig. 47 is a schematic cross-sectional view illustrating a fluid chip of a ninth embodiment.
Fig. 48 is an explanatory diagram illustrating a surface pattern forming step of the ninth embodiment.
Fig. 49 is an explanatory diagram illustrating a back surface pattern forming step of the ninth embodiment.
Fig. 50 is an explanatory diagram illustrating a first etching step of the ninth embodiment.
Fig. 51 is an explanatory diagram illustrating a second etching step of the ninth embodiment.
Fig. 52 is a schematic cross-sectional view illustrating a fluid chip of a tenth embodiment.
Fig. 53 is an explanatory diagram illustrating a conductive film forming step of the tenth embodiment.
Fig. 54 is an explanatory diagram illustrating a surface pattern forming step of the tenth embodiment.
Fig. 55 is an explanatory diagram illustrating a back surface pattern forming step of the tenth embodiment.
Fig. 56 is an explanatory diagram illustrating an intra-substrate flow path forming step of the tenth embodiment.
Fig. 57 is a schematic cross-sectional view illustrating an analysis device in which the fluid chip of the tenth embodiment is implemented.
Fig. 58 is a schematic cross-sectional view illustrating a fluid chip of an eleventh embodiment.
Fig. 59 is a schematic cross-sectional view illustrating a fluid chip of a twelfth embodiment.
Fig. 60 is a schematic cross-sectional view illustrating a fluid chip of a thirteenth embodiment.
Fig. 61 is a schematic cross-sectional view illustrating a fluid chip of a fourteenth embodiment.
Fig. 62 is a schematic cross-sectional view illustrating a fluid chip of a fifteenth embodiment.

### Description of Embodiments

### [First Embodiment]

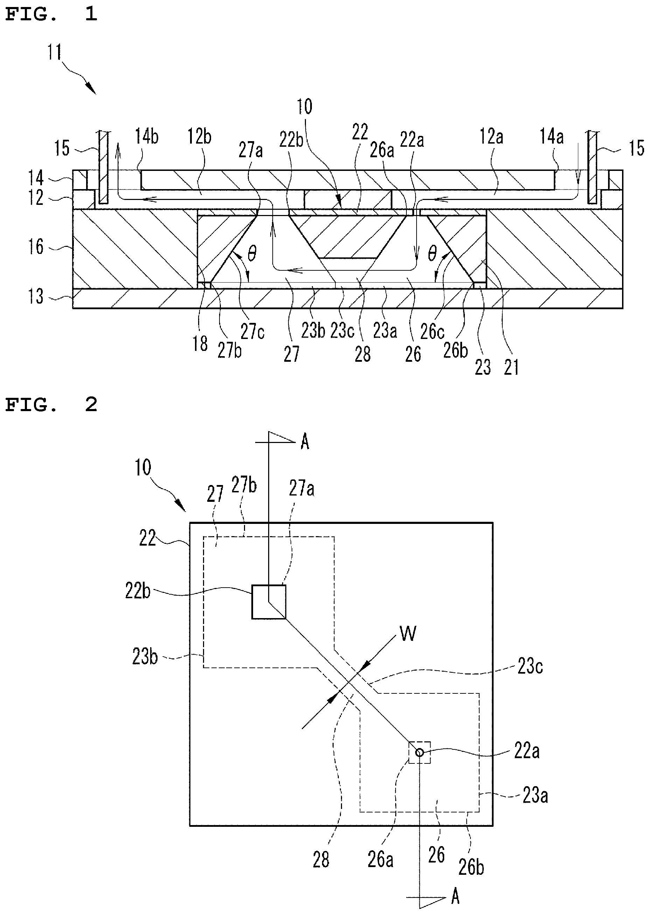

As illustrated in Fig. 1, a fluid chip 10 is used in an analysis device 11 for analyzing a minute amount of sample. The analysis device 11 analyzes the sample by detecting a change in current value at a time when a sample solution in which the sample is dispersed in a solution containing an electrolyte flows inside and the sample passes through the fluid chip 10. Specifically, by a voltage being applied to an electrode pair 15 (described later), an ion current flowing through the internal space of the smallest opening portion having the smallest opening area in the flow channel of the sample flowing in the analysis device 11 is generated. The electric resistance value of the smallest opening portion increases when the sample passes through the smallest opening portion. Accordingly, the value of the ion current changes in accordance with the volume of the sample passing through the smallest opening portion. The value of the ion current changes to a large extent in a case where the sample that has a large volume infiltrates into the internal space of the smallest opening portion. The value of the ion current changes to a small extent in a case where the sample that has a small volume infiltrates into the internal space of the smallest opening portion. The analysis device 11 analyzes the size of the sample, the shape of the sample, and so on based on the change in current value. The sample is deoxyribonucleic acid (DNA), protein, pollen, a virus, a cell, organic or inorganic particles, particulate matter (PM) such as PM 2.5, or the like. The sample is DNA in this example. In the present embodiment, an inflow opening portion 22a (described later) of the fluid chip 10 is the smallest opening portion. Accordingly, the analysis device 11 identifies the nucleic acid base molecule that constitutes the DNA and analyzes the base sequence of the DNA and the like by detecting a change in current value at a time when the DNA passes through the inflow opening portion 22a.

The analysis device 11 includes an upper-side flow path sheet 12, a lower-side cover sheet 13, an upper-side cover sheet 14, and the electrode pair 15 in addition to the fluid chip 10. The fluid chip 10 is provided between the upper-side flow path sheet 12 and the lower-side cover sheet 13. As will be described in detail later, the electrode pair 15 is provided in a supply portion 14a where the DNA is supplied and a collection portion 14b where the DNA is collected and the supply portion 14a and the collection portion 14b are provided in the same surface of the analysis device 11. In the present embodiment, the analysis device 11 further includes a chip frame 16 holding the fluid chip 10. The planar shape of the analysis device 11 is, for example, a rectangle. In the present embodiment, the planar shape of the analysis device 11 is a square in which the length of one side is 25 mm.

The upper-side flow path sheet 12 is provided on the surface of the fluid chip 10. Rubber, resin, or the like is used as the material of the upper-side flow path sheet 12. The upper-side flow path sheet corresponds to the "upper-side sheet" described in the claims.

The upper-side flow path sheet 12 has a first upper-side flow path 12a and a second upper-side flow path 12b. The first upper-side flow path 12a is connected to the supply portion 14a (described later) and guides the DNA supplied from the supply portion 14a to the fluid chip 10. The second upper-side flow path 12b is connected to the collection portion 14b (described later) and guides the DNA from the fluid chip 10 to the collection portion 14b. The shapes of the first upper-side flow path 12a and the second upper-side flow path 12b are not particularly limited. For example, the first upper-side flow path 12a and the second upper-side flow path 12b are formed in a slit shape.

The lower-side cover sheet 13 is provided on the back surface of the fluid chip 10. The lower-side cover sheet 13 constitutes the lower surface of the analysis device 11. Rubber, resin, or the like is used as the material of the lower-side cover sheet 13.

The chip frame 16 has an accommodating portion 18 where the fluid chip 10 is accommodated. The accommodating portion 18 penetrates the chip frame 16 in the thickness direction. The shape of the accommodating portion 18 is formed in accordance with the outer shape of the fluid chip 10. In the present embodiment, the planar shape of the accommodating portion 18 is a square in which the length of one side is 5 mm. Resin or the like is used as the material of the chip frame 16.

The upper-side cover sheet 14 is provided on the surface of the upper-side flow path sheet 12. Rubber, resin, or the like is used as the material of the upper-side cover sheet 14. The upper-side cover sheet 14 constitutes the upper surface of the analysis device 11. The upper-side cover sheet 14 is provided with the supply portion 14a and the collection portion 14b. In other words, the supply portion 14a and the collection portion 14b are provided in the upper surface of the analysis device 11.

The electrode pair 15 is provided in the supply portion 14a and the collection portion 14b. The electrode pair 15 is connected to an electric power source (not illustrated) and a current detection device (not illustrated). The electric power source applies a voltage to the electrode pair 15. By the voltage being applied to the electrode pair 15, the DNA is electrophoresed and the DNA passes through the fluid chip 10. It should be noted that the supplied DNA may be passed through the fluid chip 10 by pressure or may be passed through the fluid chip 10 by both electrophoresis and pressure. The current detection device detects a change in current value by using the fact that the current value changes when the DNA passes through the fluid chip 10.

The fluid chip 10 will be described with reference to Figs. 1 and 2. The planar shape of the fluid chip 10 is, for example, a rectangle. In the present embodiment, the planar shape of the fluid chip 10 is a square in which the length of one side is 5 mm (see Fig. 2). The fluid chip 10 includes a substrate 21, a surface-side insulating film 22, and a back surface-side insulating film 23 (see Fig. 1).

The substrate 21 is a silicon substrate. The thickness of the substrate 21 is 775 µm in the present embodiment. An intra-substrate flow path is provided in the substrate 21. The intra-substrate flow path guides the DNA supplied to the supply portion 14a to the collection portion 14b. In the present embodiment, the intra-substrate flow path has an upstream flow path 26, a downstream flow path 27, and a back surface flow path 28 (see Fig. 1).

The upstream flow path 26 is provided on the upstream side of the intra-substrate flow path. The upstream flow path 26 penetrates the substrate 21 in the thickness direction (see Fig. 1). The upstream flow path 26 has a surface opening 26a provided in the surface of the substrate 21, a back surface opening 26b provided in the back surface of the substrate 21, and an inner wall 26c interconnecting the surface opening 26a and the back surface opening 26b. The inner wall 26c is inclined with respect to the back surface of the substrate 21. An inclination angle θ of the inner wall 26c is approximately 55°. As illustrated in Fig. 2, the planar shape of the surface opening 26a is, for example, a rectangle. In the present embodiment, the planar shape of the surface opening 26a is a square in which the length of one side is 200 µm. The planar shape of the back surface opening 26b is, for example, a rectangle. In the present embodiment, the planar shape of the back surface opening 26b is a square. In a case where the thickness of the substrate 21 is 775 µm, the length of one side of the back surface opening 26b is 1.2 mm.

The downstream flow path 27 is provided on the downstream side of the intra-substrate flow path. The downstream flow path 27 penetrates the substrate 21 in the thickness direction (see Fig. 1). The downstream flow path 27 has a surface opening 27a provided in the surface of the substrate 21, a back surface opening 27b provided in the back surface of the substrate 21, and an inner wall 27c interconnecting the surface opening 27a and the back surface opening 27b. As in the case of the inner wall 26c, the inner wall 27c is inclined with respect to the back surface of the substrate 21 and the inclination angle θ of the inner wall 27c is approximately 55°. As illustrated in Fig. 2, the planar shape of the surface opening 27a is, for example, a rectangle. In the present embodiment, the planar shape of the surface opening 27a is a square in which the length of one side is 400 µm. The planar shape of the back surface opening 27b is, for example, a rectangle. In the present embodiment, the planar shape of the back surface opening 27b is a square. In a case where the thickness of the substrate 21 is 775 µm, the length of one side of the back surface opening 27b is 1.4 mm.

The back surface flow path 28 is provided in the back surface of the substrate 21 and interconnects the upstream flow path 26 and the downstream flow path 27 (see Fig. 1). The back surface flow path 28 guides the DNA in the upstream flow path 26 to the downstream flow path 27. The depth of the back surface flow path 28 is 35 µm in the present embodiment. As illustrated in Fig. 2, a width W of the back surface flow path 28 is 50 µm in the present embodiment.

The surface-side insulating film 22 is provided on the surface of the substrate 21 (see Fig. 1). The surface-side insulating film 22 is formed of, for example, a silicon nitride film (SiN film) or a silicon oxide film (SiO film). In the present embodiment, the surface-side insulating film 22 is formed of a SiN film. The thickness of the surface-side insulating film 22 is 50 nm in the present embodiment. The surface-side insulating film corresponds to the "insulating film" described in the claims.

The surface-side insulating film 22 is provided with the inflow opening portion 22a and an outflow opening portion 22b. In the present embodiment, the inflow opening portion 22a and the outflow opening portion 22b are provided in the thickness direction of the fluid chip 10. The inflow opening portion 22a and the outflow opening portion 22b are interconnected via the intra-substrate flow path of the substrate 21.

The inflow opening portion 22a is provided between the first upper-side flow path 12a and the upstream flow path 26 and allows the DNA in the first upper-side flow path 12a to flow into the upstream flow path 26 (see Fig. 1). In other words, the inflow opening portion 22a is provided on the upstream side of the intra-substrate flow path and allows the DNA to flow into the intra-substrate flow path. The planar shape of the inflow opening portion 22a is, for example, a circle. In the present embodiment, the diameter of the inflow opening portion 22a is 200 nm (see Fig. 2).

The outflow opening portion 22b is provided between the second upper-side flow path 12b and the downstream flow path 27 and allows the DNA in the downstream flow path 27 to flow out to the second upper-side flow path 12b (see Fig. 1). In other words, the outflow opening portion 22b is provided on the downstream side of the intra-substrate flow path and allows the DNA to flow out of the intra-substrate flow path. The planar shape of the outflow opening portion 22b is, for example, a rectangle. In the present embodiment, the planar shape of the outflow opening portion 22b is a square in which the length of one side is 400 µm (see Fig. 2).

The back surface-side insulating film 23 is provided on the back surface of the substrate 21 (see Fig. 1). In the present embodiment, the back surface-side insulating film 23 is formed of a SiN film and has a thickness of 50 nm as in the case of the surface-side insulating film 22.

An upstream-side back surface opening portion 23a, a downstream-side back surface opening portion 23b, and a connecting portion 23c are formed in the back surface-side insulating film 23 (see Fig. 1). The upstream-side back surface opening portion 23a is provided below the upstream flow path 26. The downstream-side back surface opening portion 23b is provided below the downstream flow path 27. The connecting portion 23c is provided between the upstream-side back surface opening portion 23a and the downstream-side back surface opening portion 23b and interconnects the upstream-side back surface opening portion 23a and the downstream-side back surface opening portion 23b. The planar shape of the upstream-side back surface opening portion 23a is, for example, a rectangle. In the present embodiment, the planar shape of the upstream-side back surface opening portion 23a is a square in which the length of one side is 1 mm (see Fig. 2). The planar shape of the downstream-side back surface opening portion 23b is, for example, a rectangle. In the present embodiment, the planar shape of the downstream-side back surface opening portion 23b is a square in which the length of one side is 1.4 mm. The width of the connecting portion 23c is equal to the width W of the back surface flow path 28. The width of the connecting portion 23c is 50 µm in the present embodiment.

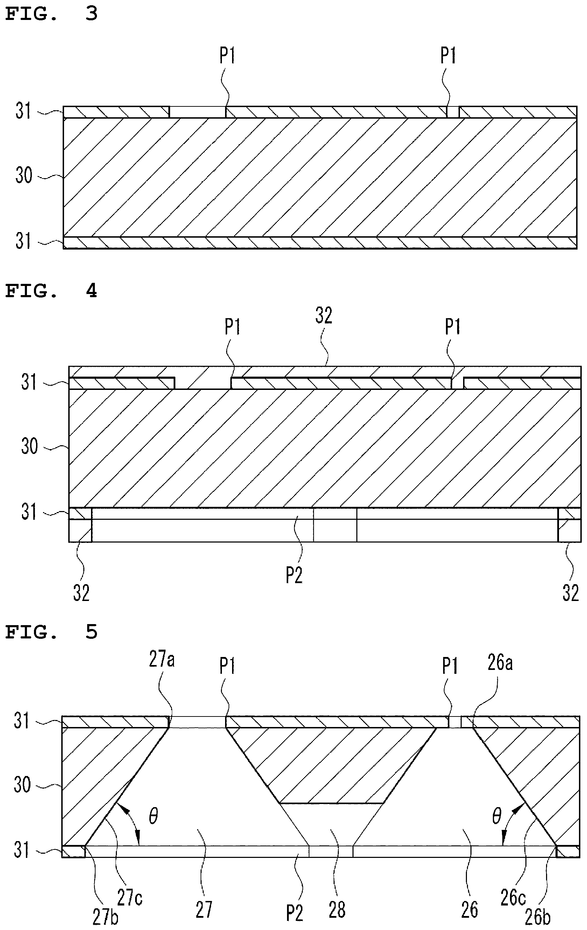

Hereinafter, a method for manufacturing the fluid chip 10 will be described with reference to Figs. 3 to 5. The fluid chip 10 is manufactured by a surface pattern forming step, a back surface pattern forming step, and an intra-substrate flow path forming step. Figs. 3 to 5 are cross-sectional views taken along line A-A of Fig. 2.

As illustrated in Fig. 3, in the surface pattern forming step, a surface pattern P1 is formed in an insulating film 31 provided on the surface of a substrate 30. A silicon substrate is used as the substrate 30. The thickness of the substrate 30 is 775 µm in the present embodiment. In the surface pattern forming step, the insulating film 31 is formed on both surfaces of the substrate 30 first. The insulating film 31 is formed by, for example, a chemical vapor deposition (CVD) method using dichlorosilane (DCS) as a source gas. Subsequently, the surface pattern P1 in which parts corresponding to the inflow opening portion 22a and the outflow opening portion 22b open is formed in the insulating film 31 provided on the surface of the substrate 30. For example, a photoresist layer (not illustrated) is formed by a photoresist being applied onto the insulating film 31 provided on the surface of the substrate 30 and the photoresist layer is patterned by the photolithography technique. Formed in the photoresist layer is a resist pattern in which parts corresponding to the inflow opening portion 22a and the outflow opening portion 22b open. The insulating film 31 on the surface of the substrate 30 is dry-etched by the photoresist layer where the resist pattern is formed being used as a mask. As a result, the surface pattern P1 is formed in the insulating film 31 on the surface side of the substrate 30. The insulating film 31 where the surface pattern P1 is formed becomes the surface-side insulating film 22 of the fluid chip 10.

As illustrated in Fig. 4, in the back surface pattern forming step, a back surface pattern P2 is formed in the insulating film 31 and a protective film 32 provided on the back surface of the substrate 30. In the back surface pattern forming step, the protective film 32 is formed on both surfaces of the substrate 30 first. The protective film 32 is preferably a material having a high etching rate selectivity with respect to a wet etching solution in anisotropic wet etching (described later). The protective film 32 is, for example, a SiO film formed by a CVD method using tetraexisilane (TEOS) as a source gas. Subsequently, the back surface pattern P2 in which parts corresponding to the back surface opening 26b of the upstream flow path 26, the back surface opening 27b of the downstream flow path 27, and the back surface flow path 28 open is formed in the insulating film 31 and the protective film 32 provided on the back surface of the substrate 30. The back surface pattern P2 is formed by, for example, a method similar to how the surface pattern P1 is formed. In other words, the back surface pattern P2 is formed by a photoresist layer (not illustrated) being formed on the protective film 32 provided on the back surface of the substrate 30, the photoresist layer being patterned by the photolithography technique, and the protective film 32 and the insulating film 31 on the back surface of the substrate 30 being sequentially dry-etched by the photoresist layer where a resist pattern is formed being used as a mask. The insulating film 31 where the back surface pattern P2 is formed becomes the back surface-side insulating film 23 of the fluid chip 10.

As illustrated in Fig. 5, the intra-substrate flow path is formed in the substrate 30 in the intra-substrate flow path forming step. In the present embodiment, the upstream flow path 26, the downstream flow path 27, and the back surface flow path 28 are formed as the intra-substrate flow path. In the intra-substrate flow path forming step, anisotropic wet etching is performed by the substrate 30 being immersed in a wet etching solution after the back surface pattern forming step. Used as the wet etching solution is an alkaline aqueous solution such as potassium hydroxide (KOH) and tetramethylammonium hydroxide (TMAH).

The protective film 32 formed on the back surface of the substrate 30 functions as a mask for anisotropic wet etching. Accordingly, the surface of the substrate 30 entirely covered with the protective film 32 is not etched and a part of the back surface of the substrate 30 exposed by the protective film 32 where the back surface pattern P2 is formed is etched. By the anisotropic wet etching being performed, the upstream flow path 26, the downstream flow path 27, and the back surface flow path 28 are formed in the substrate 30. The inclination angle θ of the inner walls 26c and 27c is determined based on the difference between the etching rates of the silicon crystal surfaces. The inclination angle θ is approximately 55° in the case of the present embodiment. It is difficult for the wet etching solution in the anisotropic wet etching to enter the small-opening width part of the protective film 32 where the back surface pattern P2 is formed. In the present embodiment, the opening width of the protective film 32 at the part that corresponds to the back surface flow path 28 is smaller than the opening width of the protective film 32 that corresponds to the back surface opening 26b of the upstream flow path 26 and the back surface opening 27b of the downstream flow path 27. Accordingly, the back surface flow path 28 has a depth at which the substrate 30 is not penetrated in the thickness direction. The depth of the back surface flow path 28 is controlled by the opening width of the protective film 32 at the part that corresponds to the back surface flow path 28 being adjusted. On the other hand, the upstream flow path 26 and the downstream flow path 27 penetrate the substrate 30 in the thickness direction. The protective film 32 is formed on the entire surface of the substrate 30. As a result, damage to the surface is suppressed and etching from the surface is prevented during the anisotropic wet etching. The substrate 30 in which the upstream flow path 26, the downstream flow path 27, and the back surface flow path 28 are formed becomes the substrate 21 of the fluid chip 10. The fluid chip 10 is obtained by the protective film 32 being removed after the anisotropic wet etching. The protective film 32 is removed by, for example, wet etching using hydrofluoric acid (HF) as a wet etching solution.

The analysis device 11 is produced by the lower-side cover sheet 13, the upper-side flow path sheet 12, and the upper-side cover sheet 14 being affixed to the fluid chip 10 manufactured through the above steps and the electrode pair 15 being provided in the supply portion 14a and the collection portion 14b. In the analysis device 11, the DNA supplied to the supply portion 14a sequentially flows through the first upper-side flow path 12a, the fluid chip 10, and the second upper-side flow path 12b and is collected in the collection portion 14b (see Fig. 1). The fluid chip 10 allows the DNA that has flowed into the inflow opening portion 22a from the first upper-side flow path 12a to sequentially pass through the upstream flow path 26, the back surface flow path 28, and the downstream flow path 27 as an intra-substrate flow path and flow out from the outflow opening portion 22b to the second upper-side flow path 12b. In this manner, the analysis device 11 forms a flow channel for passing the DNA between the supply portion 14a and the collection portion 14b. In the analysis device 11, it is preferable that the opening area of the inflow opening portion 22a, which is the smallest opening portion in the flow channel through which the DNA flows, is sufficiently smaller than the opening area of each flow path other than the inflow opening portion 22a. In this manner, the difference between the electric resistance value of the inflow opening portion 22a and the electric resistance value of the flow path other than the inflow opening portion 22a becomes sufficiently large and the analysis can be performed in a more reliable manner.

As described above, in the fluid chip 10, the inflow opening portion 22a connected to the supply portion 14a and the outflow opening portion 22b connected to the collection portion 14b are provided in the same surface of the substrate 21. As a result, the electrode pair 15 provided in the supply portion 14a and the collection portion 14b can be disposed on the same surface, and thus the analysis device 11 can be reduced in size.

In the fluid chip 10, the electrode pair 15 can be disposed on the surface side of the substrate 21, and thus the electrode pair 15 can be easily aligned. In addition, in the fluid chip 10, the upstream flow path 26 and the downstream flow path 27 are interconnected by the back surface flow path 28, and thus there is no need to provide a separate member for interconnecting the upstream flow path 26 and the downstream flow path 27. Accordingly, the fluid chip 10 itself can be reduced in size.

It should be noted that the back surface pattern P2 may be formed in the insulating film 31 on the back surface of the substrate 30 and the protective film 32 may not be formed on the back surface of the substrate 30 in a case where the insulating film 31 functions as a mask material for anisotropic wet etching although the protective film 32 where the back surface pattern P2 is formed is used as a mask for anisotropic wet etching in this example.

The diameter of the inflow opening portion 22a may be appropriately changed depending on the sample. The planar shape of the inflow opening portion 22a is not limited to the circle and may be an ellipse, a rectangle, a polygon, or the like.

The inclination angle θ of the inner wall 26c and the inner wall 27c is not limited to 55°. The inclination angle can be set within the range of 0° < θ < 180°. The inner wall 26c and the inner wall 27c are not limited to being planar and may be curved.

### [Second Embodiment]

In the first embodiment described above, the upstream flow path 26 and the downstream flow path 27 are interconnected by the back surface flow path 28. In a second embodiment, the upstream flow path 26 and the downstream flow path 27 are interconnected by a separately provided flow path sheet. In the following description, the same members as in the first embodiment are denoted by the same reference numerals and description thereof is omitted.

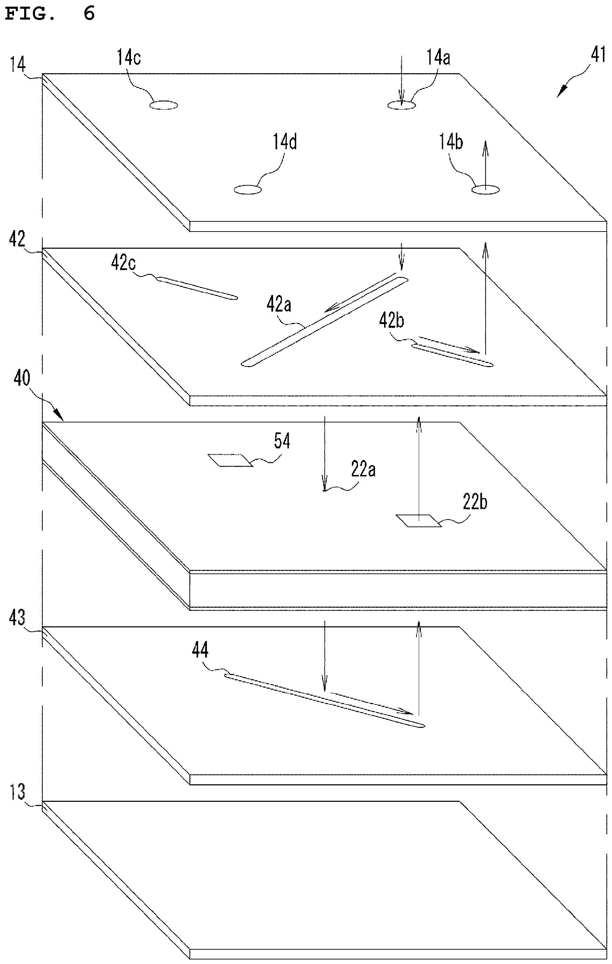

As illustrated in Fig. 6, a fluid chip 40 is provided in an analysis device 41. The analysis device 41 includes the upper-side cover sheet 14, an upper-side flow path sheet 42, a lower-side flow path sheet 43, and the lower-side cover sheet 13 in addition to the fluid chip 40. In the present embodiment, one side has a length of 25 mm in the planar shape of the analysis device 41. In the second embodiment, the upper-side cover sheet 14 is provided with a pair of the supply portion 14a and a supply portion 14d at one diagonal position and a pair of the collection portion 14b and a collection portion 14c at the other diagonal position. Although not illustrated, the analysis device 41 includes the electrode pair 15 and the electrode pair 15 is provided in, for example, the supply portion 14a and the collection portion 14b.

The upper-side flow path sheet 42 is provided on the surface of the fluid chip 40. Rubber, resin, or the like is used as the material of the upper-side flow path sheet 42.

The upper-side flow path sheet 42 has a first upper-side flow path 42a and second upper-side flow paths 42b and 42c. Although the shapes of the first upper-side flow path 42a and the second upper-side flow paths 42b and 42c are not particularly limited, the first upper-side flow path 42a and the second upper-side flow paths 42b and 42c are formed in a slit shape in the present embodiment. The first upper-side flow path 42a is provided in the shape of one diagonal line of the analysis device 41 and interconnects the supply portion 14a and the supply portion 14d. The second upper-side flow paths 42b and 42c are provided at an interval in the shape of the other diagonal line of the analysis device 41. The second upper-side flow path 42b is connected to the collection portion 14b. The second upper-side flow path 42c is connected to the collection portion 14c.

The lower-side flow path sheet 43 is provided on the back surface of the fluid chip 40. The lower-side flow path sheet 43 has a lower-side flow path 44. The lower-side flow path 44 is provided in the shape of the other diagonal line of the analysis device 41 and interconnects the first upper-side flow path 42a and the second upper-side flow paths 42b and 42c via the fluid chip 40. Rubber, resin, or the like is used as the material of the lower-side flow path sheet 43. Although the shape of the lower-side flowpath 44 is not particularly limited, the lower-side flow path 44 is formed in a slit shape in the present embodiment. The lower-side flow path sheet corresponds to the "lower-side sheet" described in the claims.

When the first upper-side flow path 42a is filled with the sample solution in the analysis device 41, the sample solution is injected from the supply portion 14a by, for example, the supply portion 14d being used as an air vent hole. The first upper-side flow path 42a is filled with the sample solution as a result. On the other hand, when the second upper-side flow paths 42b and 42c and the lower-side flow path 44 are filled with the sample solution, the sample solution is injected from the collection portion 14b by, for example, the collection portion 14c being used as an air vent hole. The second upper-side flow path 42b, the lower-side flow path 44, and the second upper-side flow path 42c are sequentially filled with the sample solution as a result. When the analysis is performed, the collection portion 14c and the supply portion 14d not provided with the electrode pair 15 (not illustrated) are blocked by a sealing member (not illustrated) or the like and the flow of the sample is restricted. It should be noted that the sample solution may be injected from the supply portion 14d by the supply portion 14a being used as an air vent hole in a case where the first upper-side flow path 42a is filled with the sample solution. In a case where the second upper-side flow paths 42b and 42c and the lower-side flowpath 44 are filled with the sample solution, the sample solution may be injected from the collection portion 14c by the collection portion 14b being used as an air vent hole. The electrode pair 15 is not limited to being provided in the supply portion 14a and the collection portion 14b. The electrode pair 15 may be provided in any of the supply portion 14a and the collection portion 14c, the supply portion 14d and the collection portion 14b, and the supply portion 14d and the collection portion 14c.

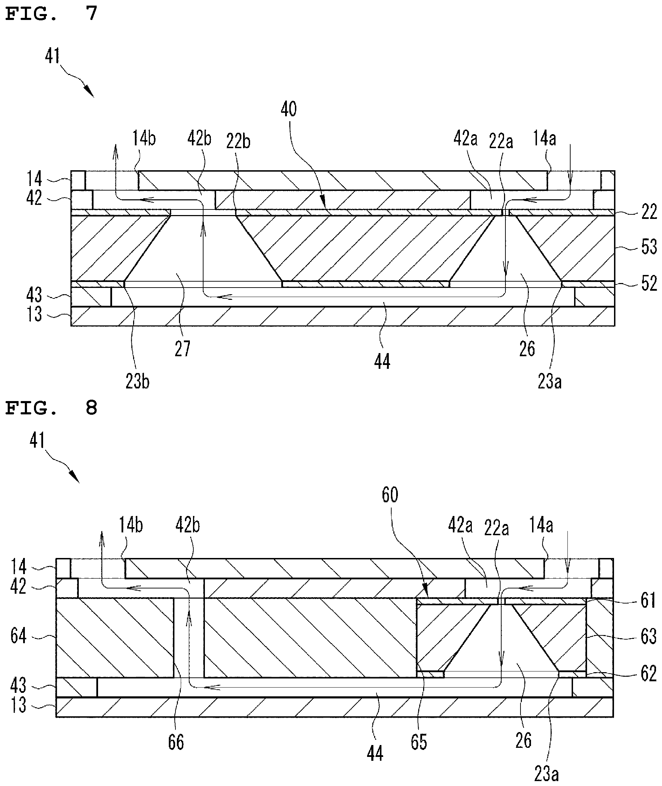

As illustrated in Fig. 7, the fluid chip 40 includes the surface-side insulating film 22, a back surface-side insulating film 52, and a substrate 53. The back surface-side insulating film 52 has the upstream-side back surface opening portion 23a and the downstream-side back surface opening portion 23b. The back surface-side insulating film 52 is different from the back surface-side insulating film 23 of the first embodiment in that the back surface-side insulating film 52 does not have the connecting portion 23c. The substrate 53 has the upstream flow path 26 and the downstream flow path 27 as an intra-substrate flow path. The substrate 53 is different from the substrate 21 of the first embodiment in that the substrate 53 does not have the back surface flow path 28. It should be noted that the fluid chip 40 is provided with a connection hole 54 (see Fig. 6), which is not illustrated in Fig. 7, penetrates the fluid chip 40 in the thickness direction, and interconnects the second upper-side flow path 42c and the lower-side flow path 44.

In the fluid chip 40 having the configuration described above, the DNA in the first upper-side flow path 42a passes through the inflow opening portion 22a and passes through the outflow opening portion 22b via the upstream flow path 26, the lower-side flow path 44, and the downstream flow path 27 in this order. As a result, the DNAmoves to the second upper-side flow path 42b. Accordingly, the analysis device 41 forms a flow channel for passing the DNA between the supply portion 14a and the collection portion 14b.

In the fluid chip 40, the inflow opening portion 22a and the outflow opening portion 22b are provided in the same surface of the substrate 53 as in the fluid chip 10 of the first embodiment. As a result, the electrode pair can be disposed on the same surface, and thus the analysis device 41 can be reduced in size.

It should be noted that the analysis device 41 may use a fluid chip 60 illustrated in Fig. 8 instead of the fluid chip 40. The fluid chip 60 has a surface-side insulating film 61, a back surface-side insulating film 62, and a substrate 63. The surface-side insulating film 61 has the inflow opening portion 22a. The surface-side insulating film 61 is different from the surface-side insulating film 22 of the first embodiment in that the surface-side insulating film 61 does not have the outflow opening portion 22b. The back surface-side insulating film 62 has the upstream-side back surface opening portion 23a. The back surface-side insulating film 62 is different from the back surface-side insulating film 23 of the first embodiment in that the back surface-side insulating film 62 does not have the downstream-side back surface opening portion 23b and the connecting portion 23c. The substrate 63 has the upstream flow path 26. The substrate 63 is different from the substrate 21 of the first embodiment in that the substrate 63 has neither the downstream flow path 27 nor the back surface flow path 28. In other words, the upstream flow path 26 is the intra-substrate flow path in the fluid chip 60.

The fluid chip 60 is held by a chip frame 64. The chip frame 64 has an accommodating portion 65 accommodating the fluid chip 60 and a connection hole 66 interconnecting the lower-side flow path 44 and the second upper-side flow path 42b. The accommodating portion 65 and the connection hole 66 penetrate the chip frame 64 in the thickness direction. In the fluid chip 60, the DNA in the first upper-side flow path 42a passes through the inflow opening portion 22a and moves to the second upper-side flow path 42b via the upstream flow path 26, the lower-side flow path 44, and the connection hole 66 in this order. In the fluid chip 60, the electrode pair can be disposed on the same surface as in the fluid chips 10 and 40. Accordingly, the analysis device 41 can be reduced in size.

### [Third Embodiment]

In a third embodiment, a reinforcing film for reinforcing the surface-side insulating film is provided between the surface-side insulating film and the substrate. In the third embodiment, those using the same members as in the second embodiment are denoted by the same reference numerals and description thereof is omitted. In addition, the description of the third embodiment focuses on the part where the upstream flow path 26 as an intra-substrate flow path is provided and illustration and description are omitted as to the part where the downstream flow path 27 is provided.

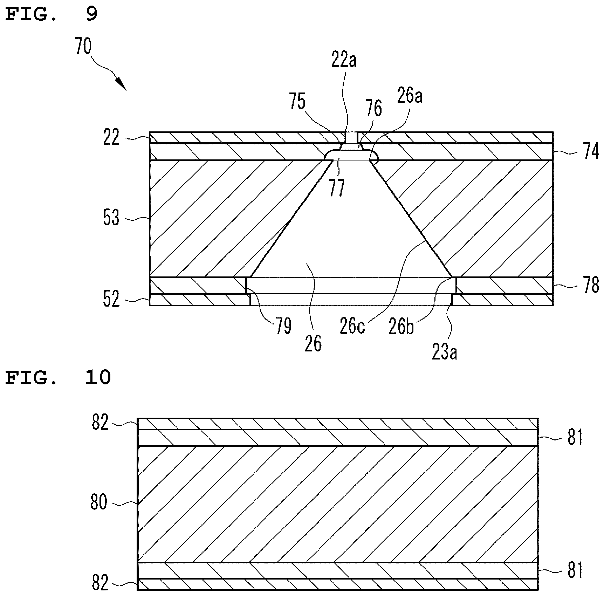

As illustrated in Fig. 9, a fluid chip 70 further includes a reinforcing film 74 provided between the surface-side insulating film 22 and the substrate 53 in addition to the surface-side insulating film 22, the back surface-side insulating film 52, and the substrate 53. The reinforcing film 74 is, for example, a thermal oxide film formed by silicon being thermally oxidized. The thickness of the reinforcing film 74 is 500 nm in the present embodiment. In the third embodiment, the diameter of the inflow opening portion 22a is 400 nm.

The reinforcing film 74 is formed with a penetrating portion 75 interconnecting the inflow opening portion 22a and the upstream flow path 26. The penetrating portion 75 has a first opening portion 76 provided in the surface of the reinforcing film 74 and a second opening portion 77 provided in the back surface of the reinforcing film 74 and penetrates the reinforcing film 74 in the thickness direction. The first opening portion 76 is connected to the inflow opening portion 22a. In this example, the inner wall surface of the first opening portion 76 is inclined with respect to the film surface of the reinforcing film 74 and the opening area of the first opening portion 76 increases from the surface of the reinforcing film 74 toward the back surface of the reinforcing film 74. The second opening portion 77 is connected to the upstream flow path 26. The first opening portion 76 is larger than the inflow opening portion 22a, and the inner wall surface is retracted from the opening end of the inflow opening portion 22a. In addition, the first opening portion 76 is smaller than the second opening portion 77. The second opening portion 77 is larger than the surface opening 26a of the upstream flow path 26 and has an inner wall surface retracted from the opening end of the surface opening 26a. The amount of retraction of the second opening portion 77 is larger than the amount of retraction of the first opening portion 76. In the present embodiment, the amount of retraction of the first opening portion 76 is 175 nm and the amount of retraction of the second opening portion 77 is 300 nm. In addition, the first opening portion 76 has a depth of 200 nm and the second opening portion 77 has a depth of 300 nm. It should be noted that the reinforcing film 74 is formed with a penetrating portion (not illustrated) interconnecting the outflow opening portion 22b and the downstream flow path 27.

In the third embodiment, the fluid chip 70 further includes a reinforcing film 78 provided between the back surface-side insulating film 52 and the substrate 53. As in the case of the reinforcing film 74, the reinforcing film 78 is a thermal oxide film formed by silicon being thermally oxidized and has a thickness of 500 nm. The reinforcing film 78 is formed with a penetrating portion 79 interconnecting the upstream-side back surface opening portion 23a and the upstream flow path 26. The penetrating portion 79 penetrates the reinforcing film 78 in the thickness direction. The penetrating portion 79 is larger than the upstream-side back surface opening portion 23a and the back surface opening 26b and has an inner wall surface retracted from the respective opening ends of the upstream-side back surface opening portion 23a and the back surface opening 26b. It should be noted that the reinforcing film 78 is formed with a penetrating portion (not illustrated) interconnecting the downstream-side back surface opening portion 23b and the downstream flow path 27.

A method for manufacturing the fluid chip 70 will be described with reference to Figs. 10 to 13. The fluid chip 70 is manufactured by a preparation step, a surface pattern forming step, a back surface pattern forming step, and an intra-substrate flow path forming step.

As illustrated in Fig. 10, in the preparation step, a reinforcing film 81 and an insulating film 82 are sequentially formed on both surfaces of a substrate 80. A silicon substrate is used as the substrate 80. The thickness of the substrate 80 is 775 µm in the present embodiment. The reinforcing film 81 is formed by, for example, a thermal CVD method in which silicon is oxidized in an oxygen atmosphere. The insulating film 82 is formed by, for example, a CVD method using DCS as a source gas.

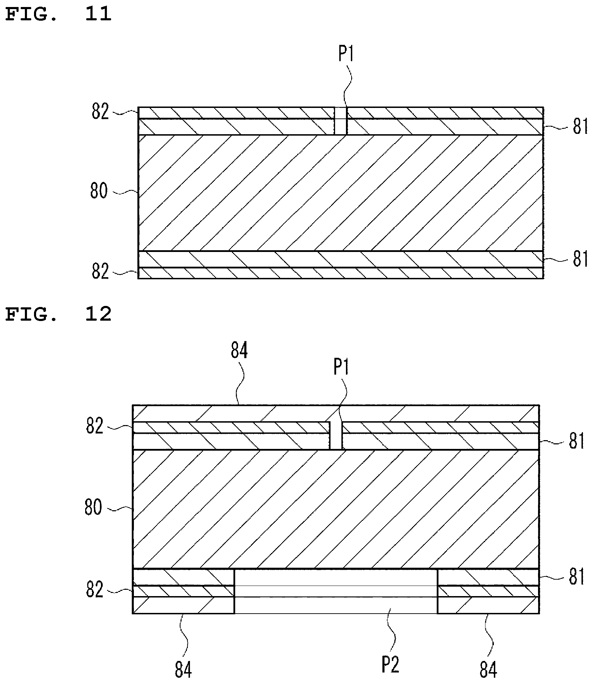

As illustrated in Fig. 11, in the surface pattern forming step, the surface pattern P1 is formed in the reinforcing film 81 and the insulating film 82 provided on the surface of the substrate 80. The surface pattern P1 is formed by, for example, a photoresist layer (not illustrated) being formed on the insulating film 82 on the surface side of the substrate 80, the photoresist layer being patterned by the photolithography technique, and the insulating film 82 and the reinforcing film 81 on the surface of the substrate 80 being sequentially dry-etched by the patterned photoresist layer being used as a mask. The reinforcing film 81 and the insulating film 82 where the surface pattern P1 is formed open at the parts that correspond to the inflow opening portion 22a and the outflow opening portion 22b. The insulating film 82 where the surface pattern P1 is formed becomes the surface-side insulating film 22 of the fluid chip 70.

As illustrated in Fig. 12, in the back surface pattern forming step, the back surface pattern P2 is formed in the reinforcing film 81, the insulating film 82, and a protective film 84 provided on the back surface of the substrate 80. In the back surface pattern forming step, the protective film 84 is formed on both surfaces of the substrate 80 first. The protective film 84 is preferably a material higher than the reinforcing film 81 in etching rate selectivity with respect to a wet etching solution in wet etching (described later) for forming the penetrating portions 75 and 79. The protective film 84 is formed by, for example, a CVD method using TEOS as a source gas. The protective film 84 is embedded in each opening of the reinforcing film 81 and the insulating film 82 where the surface pattern P1 is formed. The thickness of the protective film 84 is 500 nm in the present embodiment. After the protective film 84 is formed on both surfaces of the substrate 80, the back surface pattern P2 is formed in the reinforcing film 81, the insulating film 82, and the protective film 84 on the back surface side of the substrate 80. The back surface pattern P2 is, for example, formed similarly to the surface pattern P1. In the case of the present embodiment, the reinforcing film 81, the insulating film 82, and the protective film 84 where the back surface pattern P2 is formed open at the parts that correspond to the back surface opening 26b of the upstream flow path 26 and the back surface opening 27b of the downstream flow path 27. The insulating film 82 where the back surface pattern P2 is formed becomes the back surface-side insulating film 52 of the fluid chip 70.

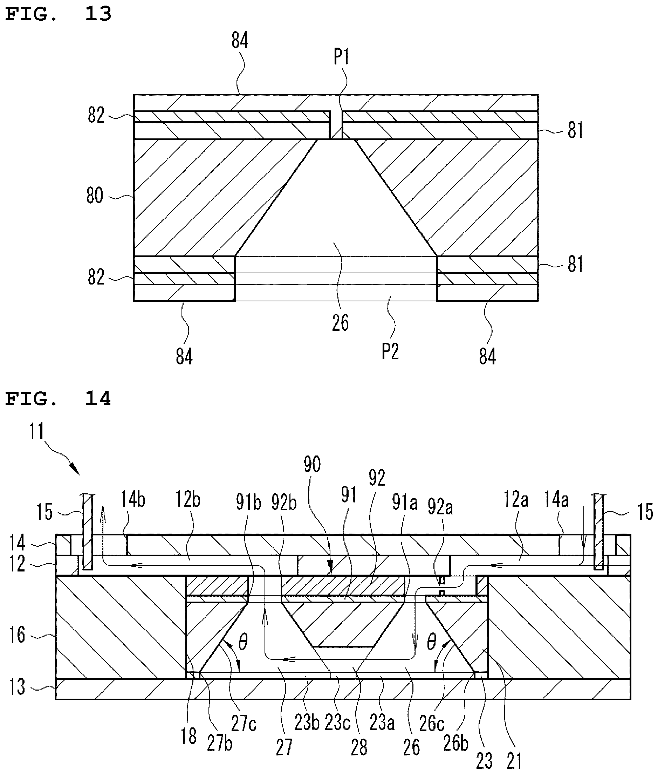

As illustrated in Fig. 13, the intra-substrate flow path is formed in the substrate 80 in the intra-substrate flow path forming step. In the case of the present embodiment, the intra-substrate flow path is the upstream flow path 26 and the downstream flow path 27 (not illustrated). Description is omitted as to the formation of the downstream flow path 27. The intra-substrate flow path is formed by anisotropic wet etching using, for example, an alkaline aqueous solution. The protective film 84 functions as a mask for anisotropic wet etching. The substrate 80 where the intra-substrate flow path is formed becomes the substrate 53 of the fluid chip 70.

After the intra-substrate flow path forming step, the protective film 84 is removed by wet etching. For example, the substrate 80 where the upstream flow path 26 is formed is immersed in the wet etching solution. The wet etching solution is HF or the like. The opening on the surface side of the substrate 80 is blocked by the protective film 84, and thus the wet etching solution flows into the upstream flow path 26 from the opening on the back surface side of the substrate 80. Apart of the reinforcing film 81 is also removed in the process of removing the protective film 84 by wet etching. The reinforcing film 81 provided on the surface of the substrate 80 is etched from the back surface side by the wet etching solution that has flowed into the upstream flow path 26. As a result of this wet etching, the protective film 84 is removed, an opening corresponding to the penetrating portion 75 is formed in the reinforcing film 81 on the surface of the substrate 80, and an opening corresponding to the penetrating portion 79 is formed in the reinforcing film 81 on the back surface of the substrate 80. The amount of retraction of the penetrating portion 75 and the penetrating portion 79 can be controlled by the thickness of the reinforcing film 81 and the etching rate selectivity of the reinforcing film 81 with respect to the wet etching solution. The reinforcing film 81 where the opening corresponding to the penetrating portion 75 is formed becomes the reinforcing film 74 of the fluid chip 70. The reinforcing film 81 where the opening corresponding to the penetrating portion 79 is formed becomes the reinforcing film 78 of the fluid chip 70. The fluid chip 70 illustrated in Fig. 9 is obtained as a result. It should be noted that it is preferable to separately perform wet etching for removing a part of the reinforcing film 81 in a case where no part of the reinforcing film 81 is removed during the wet etching of the protective film 84. The surface-side insulating film 22 is reinforced by the reinforcing film 78, and thus the fluid chip 70 has excellent durability with, for example, damage to the surface-side insulating film 22 suppressed.

It should be noted that the surface pattern P1 may be formed only in the insulating film 82 provided on the surface of the substrate 80, without being formed in the reinforcing film 81 provided on the surface of the substrate 80, in the surface pattern forming step. In this case, the opening area of the first opening portion 76 decreases from the surface side of the reinforcing film 74 toward the back surface side of the reinforcing film 74. The inner wall surface of the first opening portion 76 has, for example, a curved shape that is convex toward the outside. The amount of retraction from the opening end of the inflow opening portion 22a in the connecting portion where the first opening portion 76 and the second opening portion 77 are interconnected is smaller than the amount of retraction of the first opening portion 76. For example, the amount of retraction of the first opening portion 76 is 300 nm and the amount of retraction of the connecting portion between the first opening portion 76 and the second opening portion 77 is 230 nm.

The protective film 84 may not be formed in the back surface pattern forming step. In a case where the protective film 84 is not formed, the insulating film 82 preferably functions as a mask material for anisotropic wet etching in the intra-substrate flow path forming step. In the wet etching after the intra-substrate flow path forming step, the amount of retraction of the first opening portion 76 and the amount of retraction of the second opening portion 77 are substantially equal to each other. For example, the amount of retraction of each of the first opening portion 76 and the second opening portion 77 is 300 nm.

### [Fourth Embodiment]

In each of the embodiments described above, the inflow opening portion is provided in the thickness direction of the fluid chip. In a fourth embodiment, an inflow opening portion is provided in a direction different from the thickness direction of the fluid chip.

As illustrated in Fig. 14, a fluid chip 90 is provided in the analysis device 11 instead of the fluid chip 10 of the first embodiment. In the fluid chip 90, a surface-side insulating film 91 is provided on the surface of the substrate 21 and the back surface-side insulating film 23 is provided on the back surface of the substrate 21. The surface-side insulating film 91 is formed of, for example, a SiN film. A through hole 91a connected to the upstream flow path 26 and a through hole 91b connected to the downstream flow path 27 are formed in the surface-side insulating film 91.

An insulating film 92 is provided on the surface of the surface-side insulating film 91. The insulating film 92 is formed of, for example, a SiN film. The insulating film 92 has an inflow opening portion 92a provided between the first upper-side flow path 12a and the through hole 91a and an outflow opening portion 92b provided between the second upper-side flow path 12b and the through hole 91b. The outflow opening portion 92b is provided in the thickness direction of the fluid chip 90. The outflow opening portion 92b is similar to that in the first embodiment, and thus description thereof is omitted.

The inflow opening portion 92a is different from the inflow opening portion 22a of the first embodiment in that the inflow opening portion 92a is provided in a direction different from the thickness direction of the fluid chip 90. In this example, the inflow opening portion 92a is provided in a direction orthogonal to the thickness direction of the fluid chip 90. In the fourth embodiment, the smallest opening portion in the sample flow channel is the inflow opening portion 92a.

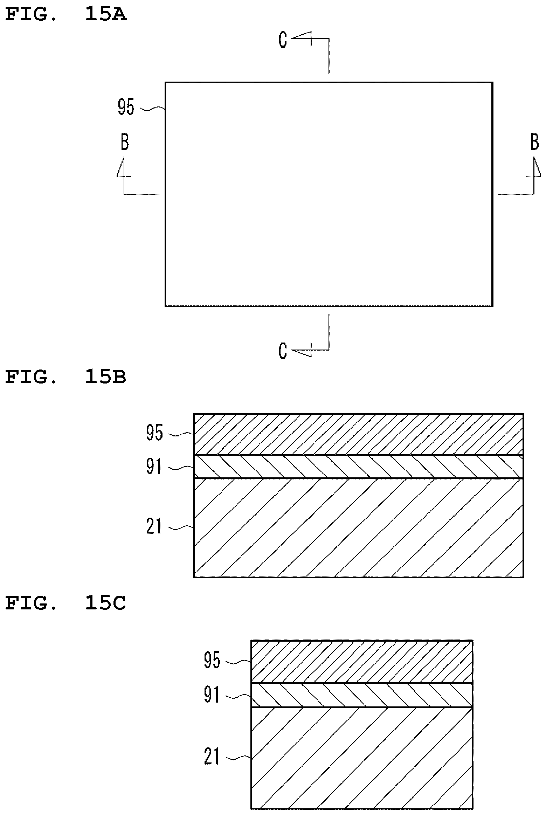

An example of a method for forming the inflow opening portion 92a will be described with reference to Figs. 15A to 15C to 19A to 19C. The following description focuses on the part of the fluid chip 90 where the inflow opening portion 92a is formed. The inflow opening portion 92a is formed by a first insulating film forming step, a second insulating film forming step, a third insulating film forming step, an insulating film processing step, and an insulating film removing step.

As illustrated in Figs. 15A to 15C, in the first insulating film forming step, a first insulating film 95 is formed on the surface of the surface-side insulating film 91 after the surface-side insulating film 91 is formed on the substrate 21. Fig. 15A is a plan view, Fig. 15B is a cross-sectional view taken along line B-B of Fig. 15A, and Fig. 15C is a cross-sectional view taken along line C-C of Fig. 15A. The surface-side insulating film 91 and the first insulating film 95 can be formed by the same method. For example, the surface-side insulating film 91 and the first insulating film 95 are formed by a CVD method using DCS as a source gas.

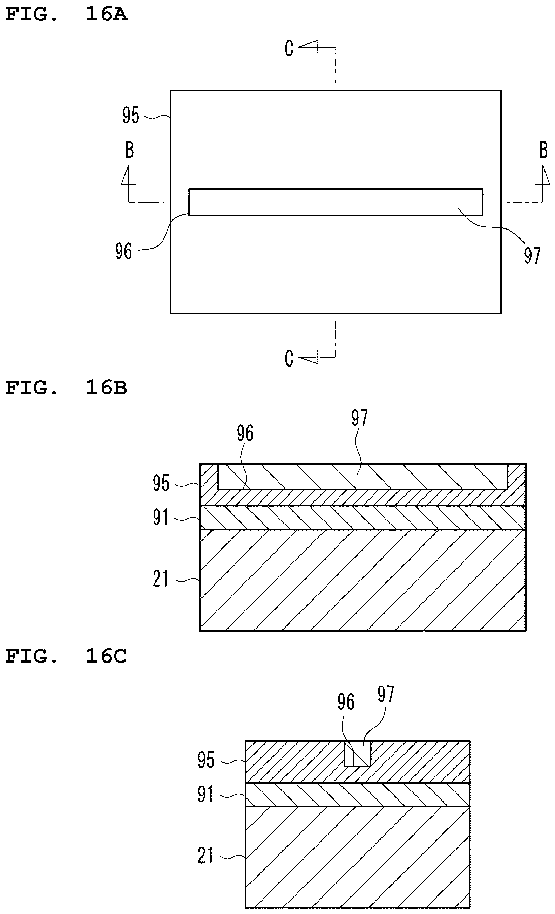

As illustrated in Figs. 16A to 16C, in the second insulating film forming step, a groove 96 is formed in the first insulating film 95 and a second insulating film 97 is formed in the groove 96. Fig. 16A is a plan view, Fig. 16B is a cross-sectional view taken along line B-B of Fig. 16A, and Fig. 16C is a cross-sectional view taken along line C-C of Fig. 16A. As illustrated in Fig. 16A, the planar shape of the groove 96 is, for example, a rectangle. The length of the short side of the groove 96 is the length of the inflow opening portion 92a in the width direction orthogonal to the thickness direction, that is, the width of the inflow opening portion 92a. The depth of the groove 96 is the length of the inflow opening portion 92a in the thickness direction, that is, the height of the inflow opening portion 92a. The groove 96 is formed by, for example, a photoresist layer (not illustrated) being formed by a photoresist being applied onto the surface of the first insulating film 95, the photoresist layer being patterned by the photolithography technique, and the first insulating film 95 being dry-etched by the photoresist layer where a resist pattern is formed being used as a mask. After the groove 96 is formed, a SiO film is formed on the entire surface of the first insulating film 95 by, for example, a CVD method using TEOS as a source gas. Next, the surface of the SiO film is flattened. A chemical mechanical polishing (CMP) device or the like is used for the flattening. It is preferable that the flattening is performed such that the surface of the first insulating film 95 is exposed. As a result of the flattening, the SiO film on the surface of the first insulating film 95 is removed and the second insulating film 97 is formed by the SiO film that remains in the groove 96.

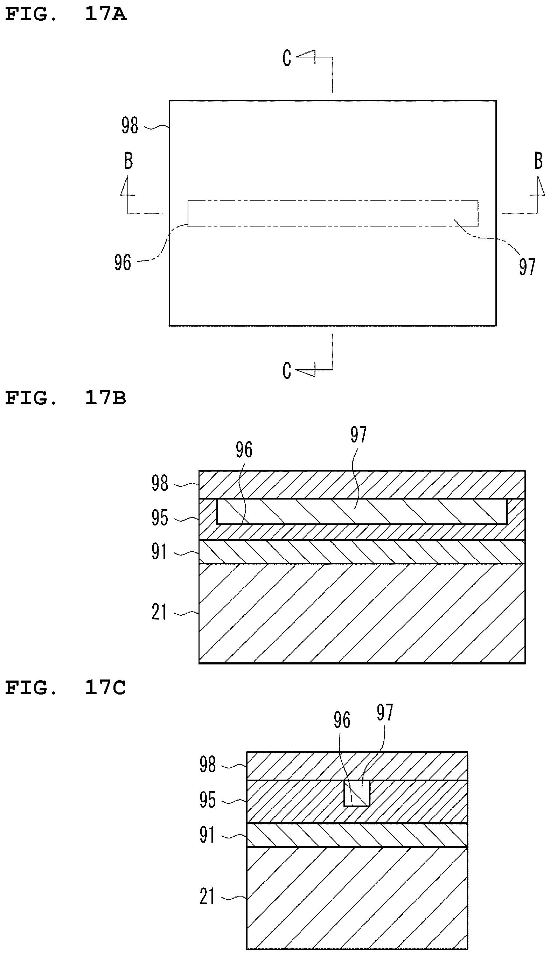

As illustrated in Figs. 17A to 17C, in the third insulating film forming step, a third insulating film 98 is formed on the surfaces of the first insulating film 95 and the second insulating film 97. Fig. 17A is a plan view, Fig. 17B is a cross-sectional view taken along line B-B of Fig. 17A, and Fig. 17C is a cross-sectional view taken along line C-C of Fig. 17A. The third insulating film 98 is formed by, for example, a CVD method using DCS as a source gas.

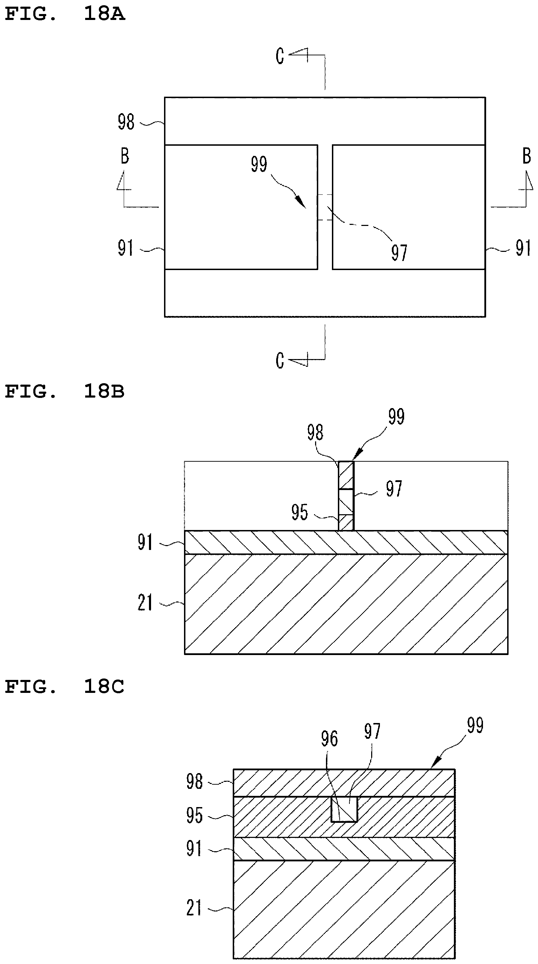

As illustrated in Figs. 18A to 18C, in the insulating film processing step, a plate-shaped body 99 is formed by the first insulating film 95, the second insulating film 97, and the third insulating film 98 being processed. Fig. 18A is a plan view, Fig. 18B is a cross-sectional view taken along line B-B of Fig. 18A, and Fig. 18C is a cross-sectional view taken along line C-C of Fig. 18A. The planar shape of the plate-shaped body 99 illustrated in Fig. 18A is a rectangle. The length of the short side of the plate-shaped body 99 is the length of the inflow opening portion 92a in a direction orthogonal to the thickness direction and the width direction, that is, the length of the inflow opening portion 92a. Dry etching or the like is performed in the insulating film processing step.

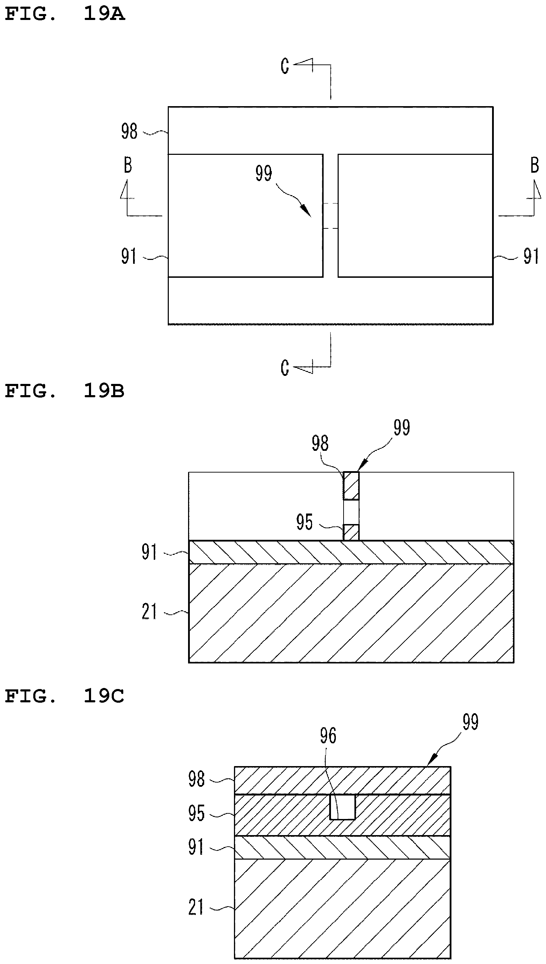

As illustrated in Figs. 19A to 19C, the second insulating film 97 is removed from the plate-shaped body 99 in the insulating film removing step. Fig. 19A is a plan view, Fig. 19B is a cross-sectional view taken along line B-B of Fig. 19A, and Fig. 19C is a cross-sectional view taken along line C-C of Fig. 19A. Wet etching using, for example, HF as a wet etching solution is performed in the second insulating film removing step. The second insulating film 97 is removed from the plate-shaped body 99, and the first insulating film 95 and the third insulating film 98 remain. The plate-shaped body 99 that is formed by the remaining first insulating film 95 and third insulating film 98 becomes the inflow opening portion 92a. The opening area of the inflow opening portion 92a is determined by the length of the short side of the groove 96 and the depth of the groove 96. Of the length of the short side of the groove 96 and the depth of the groove 96, the depth of the groove 96 can be equal to or smaller than the minimum processing dimension of the photolithography technique. Accordingly, the fluid chip 90 is excellent in terms of the design freedom of the inflow opening portion 92a as the smallest opening portion.

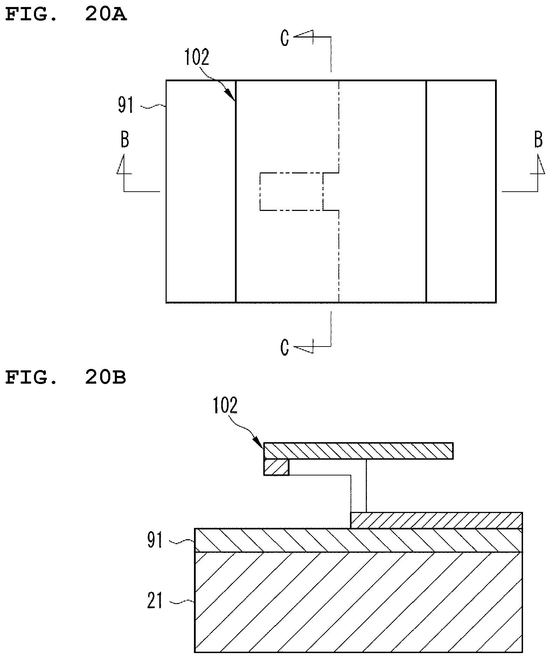

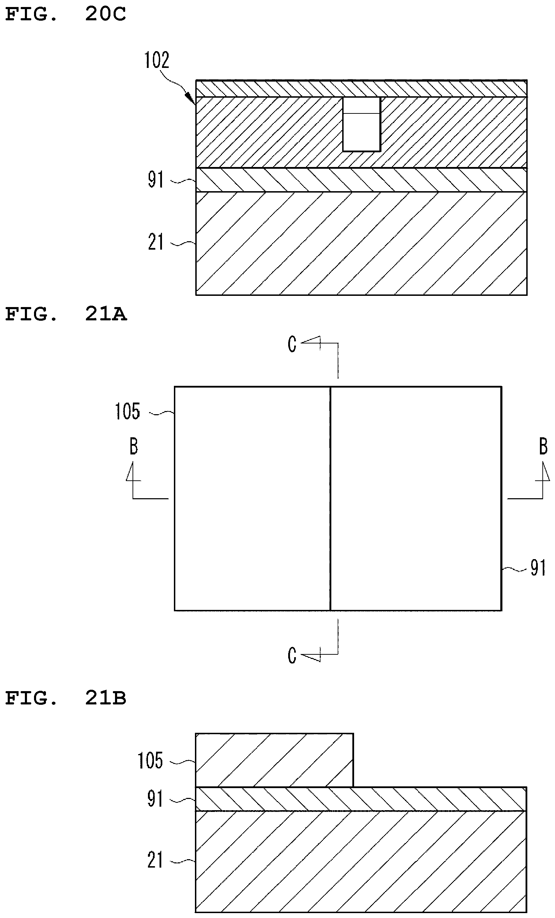

It should be noted that the fluid chip 90 may be provided with an inflow opening portion 102 illustrated in Figs. 20A to 20C instead of the inflow opening portion 92a. Fig. 20A is a plan view, Fig. 20B is a cross-sectional view taken along line B-B of Fig. 20A, and Fig. 20C is a cross-sectional view taken along line C-C of Fig. 20A. A method for forming the inflow opening portion 102 will be described with reference to Figs. 21A to 21C to 27A to 27C. The inflow opening portion 102 is formed by a first insulating film forming step, a second insulating film forming step, a third insulating film forming step, a groove forming step, a fourth insulating film forming step, a fifth insulating film forming step, an insulating film processing step, and an insulating film removing step.

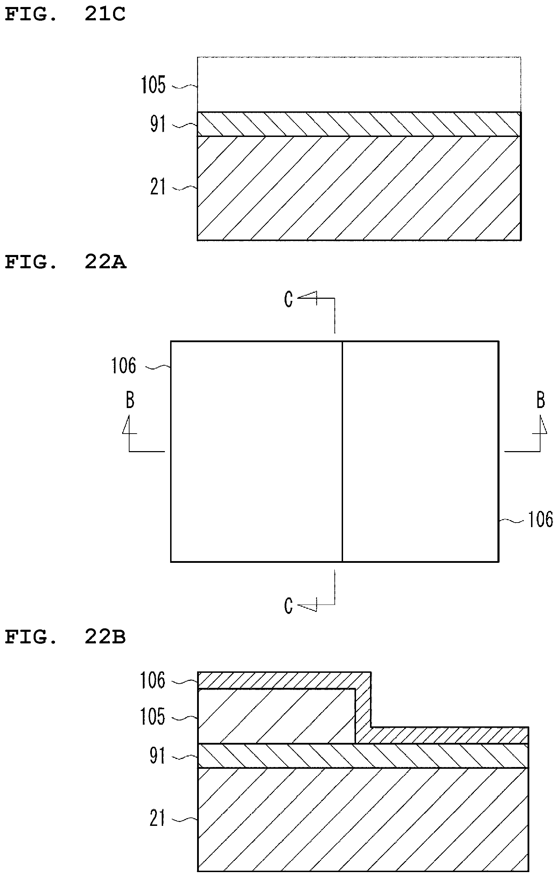

As illustrated in Figs. 21A to 21C, a first insulating film 105 is formed on the surface of the surface-side insulating film 91 in the first insulating film forming step. Fig. 21A is a plan view, Fig. 21B is a cross-sectional view taken along line B-B of Fig. 21A, and Fig. 21C is a cross-sectional view taken along line C-C of Fig. 21A. In the first insulating film forming step, a SiO film is formed on the entire surface of the surface-side insulating film 91 by, for example, a CVD method using TEOS as a source gas and a part of the SiO film is removed by dry etching. The first insulating film 105 is formed as a result.

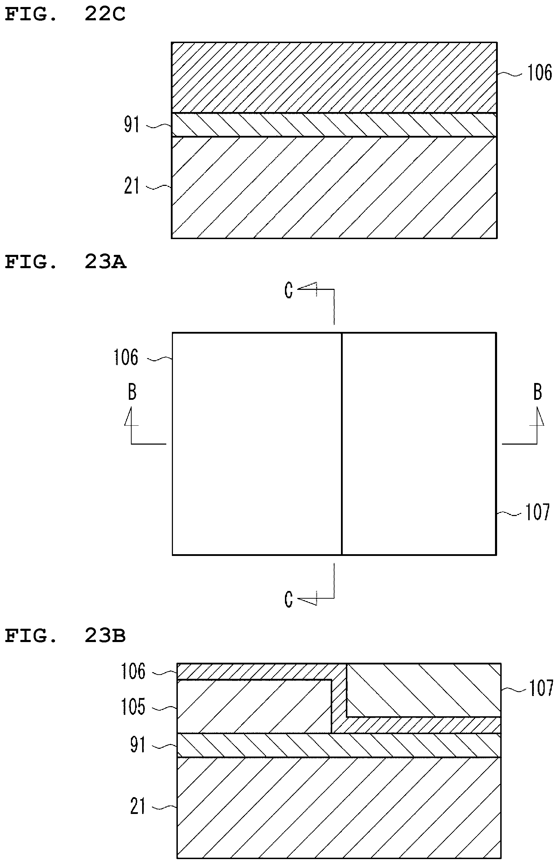

As illustrated in Figs. 22A to 22C, a second insulating film 106 is formed on the surface of the surface-side insulating film 91 and the surface of the first insulating film 105 in the second insulating film forming step. Fig. 22A is a plan view, Fig. 22B is a cross-sectional view taken along line B-B of Fig. 22A, and Fig. 22C is a cross-sectional view taken along line C-C of Fig. 22A. The second insulating film 106 is formed by, for example, a CVD method using DCS as a source gas. The second insulating film 106 is formed in a step shape. The part of the second insulating film 106 that is formed on the surface of the surface-side insulating film 91 is lower by one step than the part of the second insulating film 106 that is formed on the surface of the first insulating film 105.

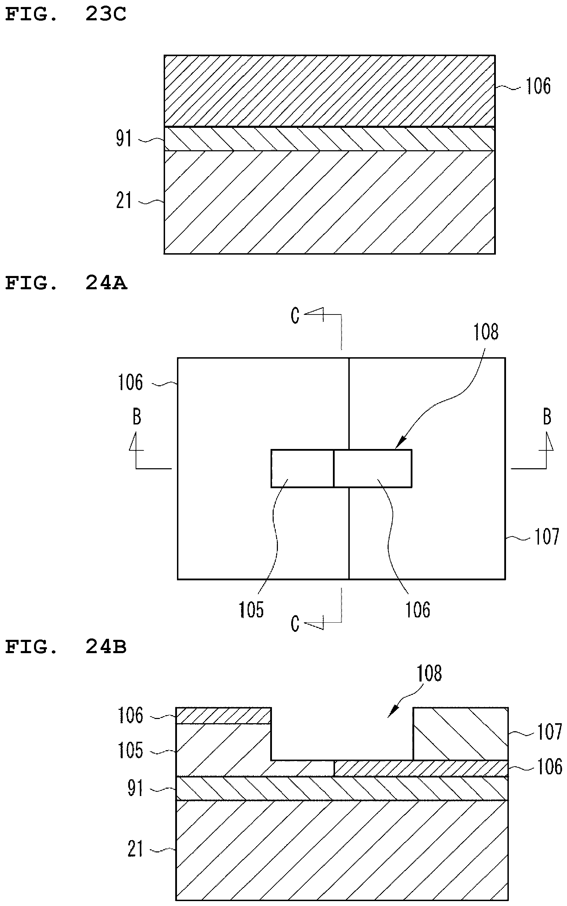

As illustrated in Figs. 23A to 23C, a third insulating film 107 is formed on the surface of the second insulating film 106 in the third insulating film forming step. Fig. 23A is a plan view, Fig. 23B is a cross-sectional view taken along line B-B of Fig. 23A, and Fig. 23C is a cross-sectional view taken along line C-C of Fig. 23A. In the third insulating film forming step, a SiO film is formed on the entire surface of the second insulating film 106 by, for example, a CVD method using TEOS as a source gas, the surface of the SiO film is flattened by means of a CMP device, and the third insulating film 107 is formed as a result. As a result of the flattening, the part of the second insulating film 106 that is formed on the surface of the first insulating film 95 is exposed. The third insulating film 107 is formed by the SiO film that remains at the part of the second insulating film 106 formed on the surface of the surface-side insulating film 91.

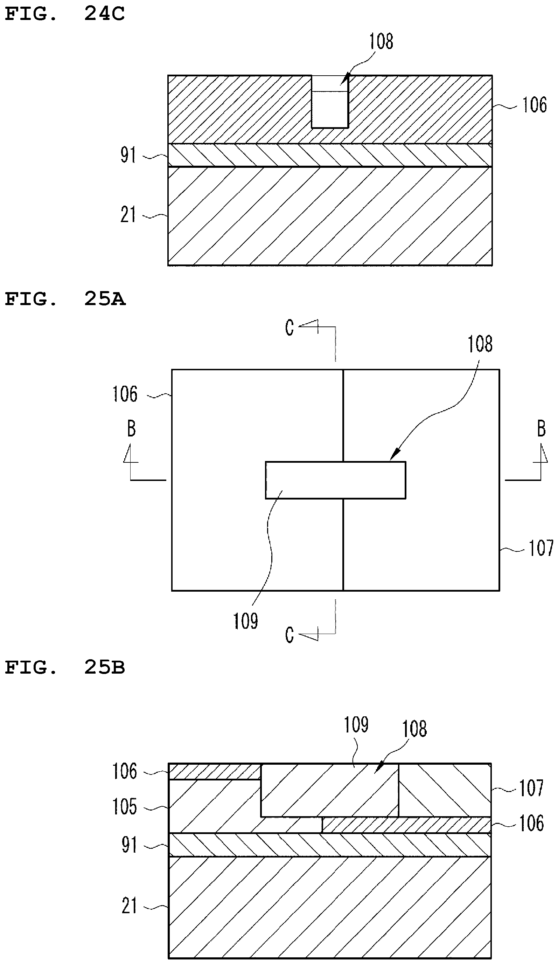

As illustrated in Figs. 24A to 24C, in the groove forming step, a groove 108 is formed by the first insulating film 105, the second insulating film 106, and the third insulating film 107 being partially removed. Fig. 24A is a plan view, Fig. 24B is a cross-sectional view taken along line B-B of Fig. 24A, and Fig. 24C is a cross-sectional view taken along line C-C of Fig. 24A. Dry etching or the like is performed in the groove forming step.

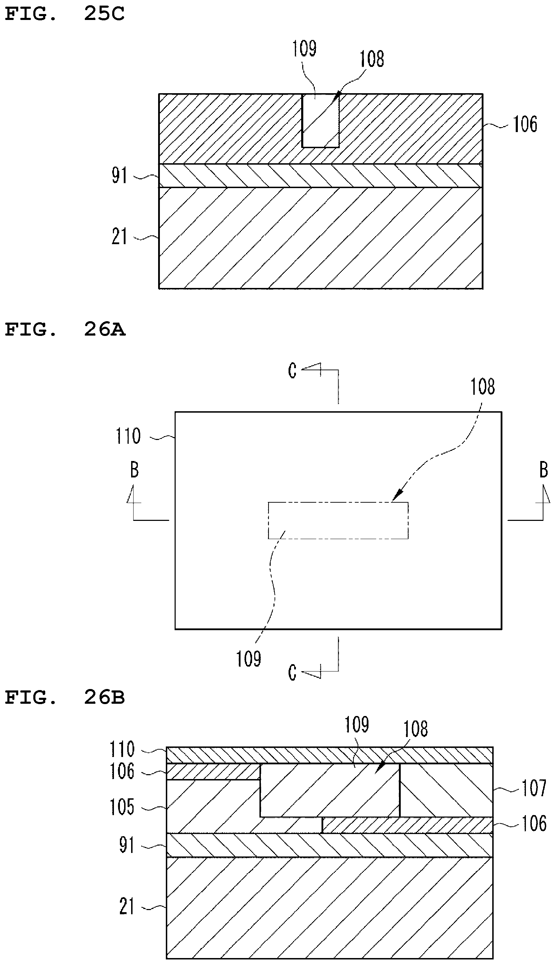

As illustrated in Figs. 25A to 25C, a fourth insulating film 109 is formed in the groove 108 in the fourth insulating film forming step. Fig. 25A is a plan view, Fig. 25B is a cross-sectional view taken along line B-B of Fig. 25A, and Fig. 25C is a cross-sectional view taken along line C-C of Fig. 25A. In the fourth insulating film forming step, the fourth insulating film 109 is formed in the groove 108 by, for example, a CVD method using TEOS as a source gas and flattening by means of a CMP device. The fourth insulating film 109 is formed of a SiO film.

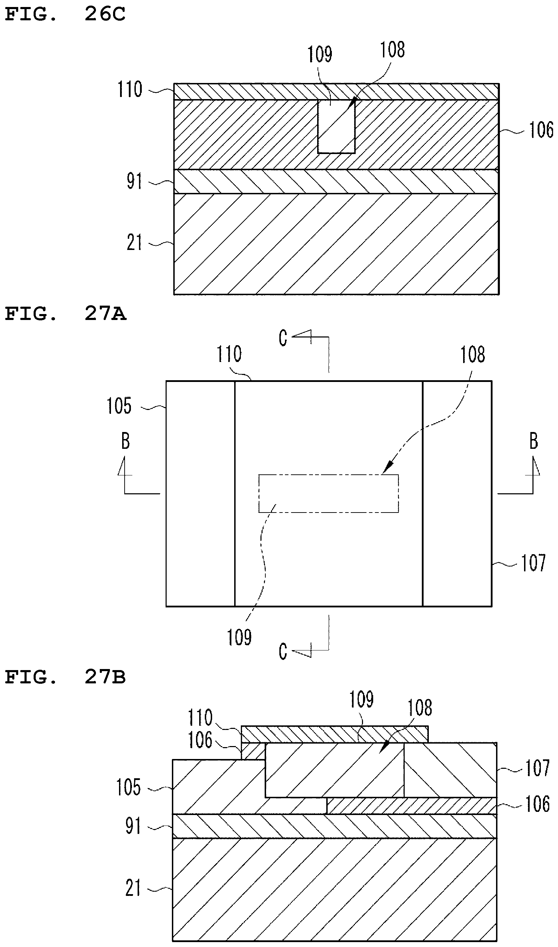

As illustrated in Figs. 26A to 26C, in the fifth insulating film forming step, a fifth insulating film 110 is formed on the flat surface that is formed by the second insulating film 106, the third insulating film 107, and the fourth insulating film 109. Fig. 26A is a plan view, Fig. 26B is a cross-sectional view taken along line B-B of Fig. 26A, and Fig. 26C is a cross-sectional view taken along line C-C of Fig. 26A. The fifth insulating film 110 is formed by, for example, a CVD method using DCS as a source gas.

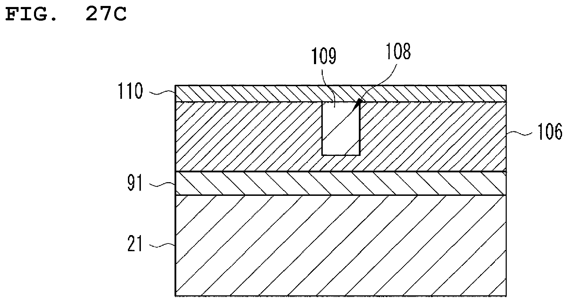

As illustrated in Figs. 27A to 27C, in the insulating film processing step, the second insulating film 106 and the fifth insulating film 110 are processed and a part of the first insulating film 105 formed below the second insulating film 106 and a part of the third insulating film 107 formed below the fifth insulating film 110 are exposed. Fig. 27A is a plan view, Fig. 27B is a cross-sectional view taken along line B-B of Fig. 27A, and Fig. 27C is a cross-sectional view taken along line C-C of Fig. 27A. In the insulating film processing step, the second insulating film 106 and the fifth insulating film 110 are processed by, for example, dry etching.

The first insulating film 105, the third insulating film 107, and the fourth insulating film 109 are removed in the insulating film removing step. In this example, the first insulating film 105, the third insulating film 107, and the fourth insulating film 109 are formed of SiO. Accordingly, wet etching using HF as a wet etching solution is performed in the insulating film removing step. The inflow opening portion 102 illustrated in Figs. 20A to 20C is formed by the second insulating film 106 and the fifth insulating film 110 that remain after the insulating film removing step. In the fluid chip 90 that has the inflow opening portion 102, the groove 108 is capable of having a depth equal to or smaller than the minimum processing dimension of the photolithography technique, and thus the fluid chip 90 is excellent in terms of the design freedom of the inflow opening portion 102 as the smallest opening portion.

### [Fifth Embodiment]

In each of the embodiments described above, the electrode pair 15 is provided on the upper surface of the analysis device 11 or 41. In a fifth embodiment, the electrode pair 15 is provided on the side surface of an analysis device.

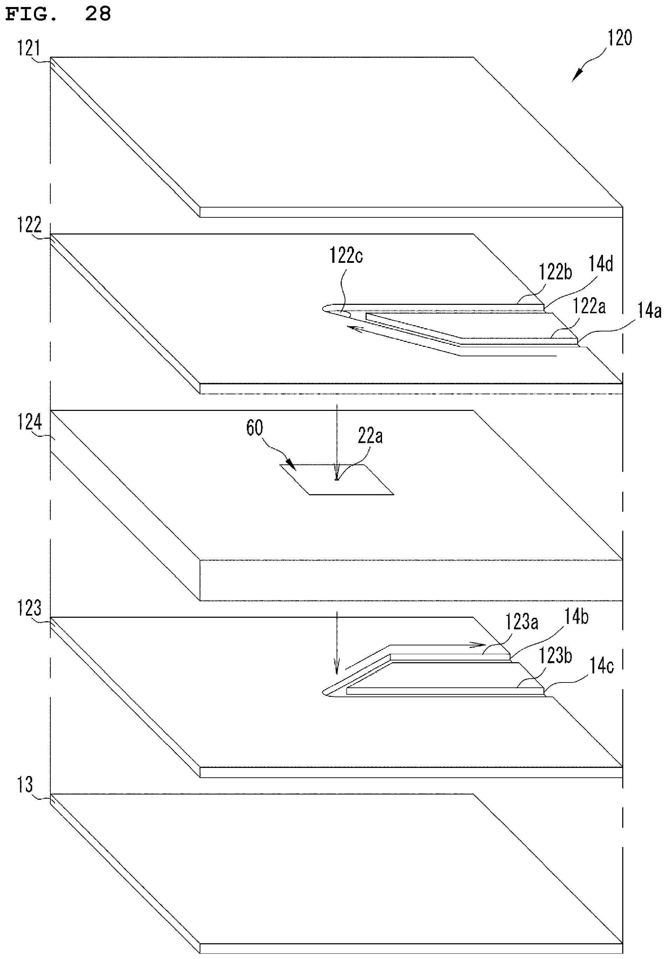

As illustrated in Fig. 28, the fluid chip 60 is used in an analysis device 120. In the fifth embodiment, the fluid chip 60 is held by a chip frame 124. The chip frame 124 is different from the chip frame 64 of the second embodiment in that the chip frame 124 does not have the connection hole 66. The analysis device 120 includes an upper-side cover sheet 121, an upper-side flow path sheet 122, a lower-side flow path sheet 123, and the lower-side cover sheet 13. In addition, the analysis device 120 has the pair of supply portions 14a and 14d and the pair of collection portions 14b and 14c as in the case of the analysis device 41 of the second embodiment.

The upper-side cover sheet 121 is different from the upper-side cover sheet 14 of the second embodiment in that the upper-side cover sheet 121 is not provided with the pair of supply portions 14a and 14d and the pair of collection portions 14b and 14c.

The upper-side flow path sheet 122 has the pair of supply portions 14a and 14d, a first upper-side flow path 122a, a second upper-side flow path 122b, and an opening portion 122c. The pair of supply portions 14a and 14d are provided in the side surface of the upper-side flow path sheet 122. The supply portion 14a is connected to one end of the first upper-side flow path 122a. The supply portion 14d is connected to one end of the second upper-side flow path 122b. The first upper-side flow path 122a and the second upper-side flow path 122b are grooves formed in the surface of the upper-side flow path sheet 122. The other end of the first upper-side flow path 122a and the other end of the second upper-side flow path 122b are connected to each other. The opening portion 122c is provided at the part where the first upper-side flow path 122a and the second upper-side flow path 122b are interconnected and is connected to the inflow opening portion 22a of the fluid chip 60.

The lower-side flow path sheet 123 has the pair of collection portions 14b and 14c, a first lower-side flow path 123a, and a second lower-side flow path 123b. The pair of collection portions 14b and 14c are provided in the side surface of the lower-side flow path sheet 123. The collection portion 14b is connected to one end of the first lower-side flow path 123a. The collection portion 14c is connected to one end of the second lower-side flow path 123b. The first lower-side flow path 123a and the second lower-side flow path 123b are grooves formed in the surface of the lower-side flow path sheet 123. The other end of the first lower-side flow path 123a and the other end of the second lower-side flow path 123b are connected to each other. The part where the first lower-side flow path 123a and the second lower-side flow path 123b are interconnected is connected to the intra-substrate flow path (not illustrated) of the fluid chip 60.

In the analysis device 120, the pair of supply portions 14a and 14d and the pair of collection portions 14b and 14c are provided in the same side surface. When the upper-side flow path sheet 122 is filled with the sample solution, the sample solution is injected from the supply portion 14a by, for example, the supply portion 14d being used as an air vent hole. The first upper-side flow path 122a, the second upper-side flow path 122b, and the opening portion 122c are filled with the sample solution as a result. When the lower-side flow path sheet 123 is filled with the sample solution, the sample solution is injected from the collection portion 14b by the collection portion 14c being used as an air vent hole. The first lower-side flow path 123a and the second lower-side flow path 123b are filled with the sample solution as a result. When the analysis is performed, the electrode pair 15 (not illustrated) is provided in, for example, the supply portion 14a and the collection portion 14b, the supply portion 14d and the collection portion 14c are blocked by a sealing member (not illustrated) or the like, and the flow of the sample is restricted. It should be noted that the supply portion 14a and the collection portion 14b provided with the electrode pair 15 may be provided on the same surface and the positions of the supply portion 14d and the collection portion 14c not provided with the electrode pair 15 are not particularly limited as for the pair of supply portions 14a and 14d and the pair of collection portions 14b and 14c.

It should be noted that the analysis device 120 is not limited to the case where the fluid chip 60 is used and the analysis device 120 may use the fluid chips 10, 40, 70, and 90. In this case, the upper-side flow path sheet 122 is provided with a supply portion and a collection portion. Further, two systems of grooves are formed in the surface of the upper-side flow path sheet 122, one being an upper-side flow path connected to the supply portion and the other being an upper-side flow path connected to the collection portion.

The pair of supply portions 14a and 14d may be provided in the side surface of the upper-side cover sheet 121 instead of being provided in the side surface of the upper-side flow path sheet 122. The pair of collection portions 14b and 14c may be provided in the side surface of the lower-side cover sheet 13 instead of being provided in the side surface of the lower-side flow path sheet 123.

### [Sixth Embodiment]

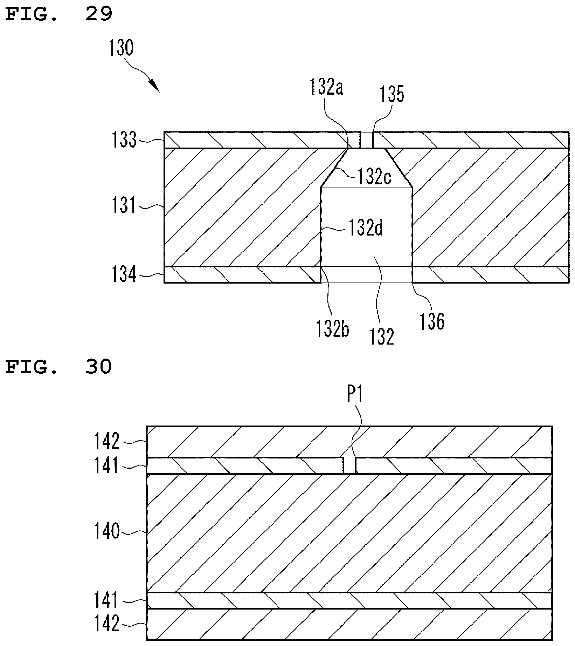

As illustrated in Fig. 29, a fluid chip 130 is used instead of the fluid chip 60 in, for example, the analysis device 41 illustrated in Fig. 8.

The fluid chip 130 includes a substrate 131, an upstream flow path 132 as an intra-substrate flow path, a surface-side insulating film 133, a back surface-side insulating film 134, an inflow opening portion 135, and a back surface opening portion 136. The substrate 131 is, for example, a silicon substrate having a thickness of 775 µm and a plane orientation of (100) . The upstream flow path 132 penetrates the substrate 131 in the thickness direction.

The surface-side insulating film 133 is provided on the surface of the substrate 131. The surface-side insulating film 133 is formed of, for example, a SiN film or a SiO film. In this example, the surface-side insulating film 133 is formed of a SiN film. The thickness of the surface-side insulating film 133 is, for example, 100 nm.

The back surface-side insulating film 134 is provided on the back surface of the substrate 131. The back surface-side insulating film 134 is formed of, for example, a SiN film or a SiO film. In this example, the back surface-side insulating film 134 is formed of a SiN film as in the case of the surface-side insulating film 133. The thickness of the back surface-side insulating film 134 is, for example, 100 nm.

The inflow opening portion 135 is provided on the upstream side of the upstream flow path 132. The inflow opening portion 135 is formed in the surface-side insulating film 133 and is connected to the upstream flow path 132. The inflow opening portion 135 allows the sample to flow into the upstream flow path 132. The planar shape of the inflow opening portion 135 is a circle in the present embodiment. The diameter of the inflow opening portion 135 is 200 nm.

The back surface opening portion 136 is provided on the downstream side of the upstream flow path 132. The back surface opening portion 136 is formed in the back surface-side insulating film 134 and is connected to the upstream flow path 132. The back surface opening portion 136 allows the sample to flow out of the upstream flow path 132. In the present embodiment, the planar shape of the back surface opening portion 136 is a square in which the length of one side is 200 µm. The diameter of the inscribed circle of the square is the diameter of the back surface opening portion 136.

The upstream flow path 132 is provided in the substrate 131. The upstream flow path 132 penetrates the substrate 131 in the thickness direction. The upstream flow path 132 has a surface opening 132a, a back surface opening 132b, a first inner wall 132c, and a second inner wall 132d.

The surface opening 132a is provided in the surface of the substrate 131. The surface opening 132a is connected to the inflow opening portion 135. The planar shape of the surface opening 132a is a circle or a polygon. In the present embodiment, the planar shape of the surface opening 132a is a square in which the length of one side is 40 µm. The diameter of the inscribed circle of the square is the diameter of the surface opening 132a.

The back surface opening 132b is provided in the back surface of the substrate 131. The back surface opening 132b is connected to the back surface opening portion 136. The planar shape of the back surface opening 132b is a circle or a polygon. In the present embodiment, the planar shape of the back surface opening 132b is a square in which the length of one side is 200 µm. The diameter of the inscribed circle of the square is the diameter of the back surface opening 132b.

The first inner wall 132c is provided between the surface opening 132a and the back surface opening 132b. The upper end of the first inner wall 132c is connected to the surface opening 132a. The lower end of the first inner wall 132c is connected to the upper end of the second inner wall 132d (described later) . The first inner wall 132c is inclined with respect to the back surface of the substrate 131. The inclination angle of the first inner wall 132c is approximately 55°.

The second inner wall 132d is provided downstream of the first inner wall 132c between the surface opening 132a and the back surface opening 132b. The upper end of the second inner wall 132d is connected to the lower end of the first inner wall 132c. The lower end of the second inner wall 132d is connected to the back surface opening 132b. The second inner wall 132d is formed so as to be perpendicular to the back surface of the substrate 131.

Hereinafter, a method for manufacturing the fluid chip 130 will be described with reference to Figs. 30 to 35. The fluid chip 130 is manufactured by a surface pattern forming step, a back surface pattern forming step, and an intra-substrate flow path forming step.

As illustrated in Fig. 30, in the surface pattern forming step, the surface pattern P1 is formed in an insulating film 141 provided on the surface of a substrate 140. The substrate 140 is a silicon substrate. The thickness of the substrate 140 is 775 µm. In the surface pattern forming step, the insulating film 141 is formed on both surfaces of the substrate 140 first. The insulating film 141 is formed by, for example, a low pressure-CVD (LP-CVD) method using DCS as a source gas. Subsequently, the surface pattern P1 in which a part corresponding to the inflow opening portion 135 opens is formed in the insulating film 141 provided on the surface of the substrate 140. The specific method for forming the surface pattern P1 is similar to that in each of the embodiments described above, and thus description thereof is omitted. In the surface pattern forming step, a protective film 142 is formed on both surfaces of the substrate 140 after the surface pattern P1 is formed. The protective film 142 is, for example, a SiO film formed by a CVD method using TEOS as a source gas.

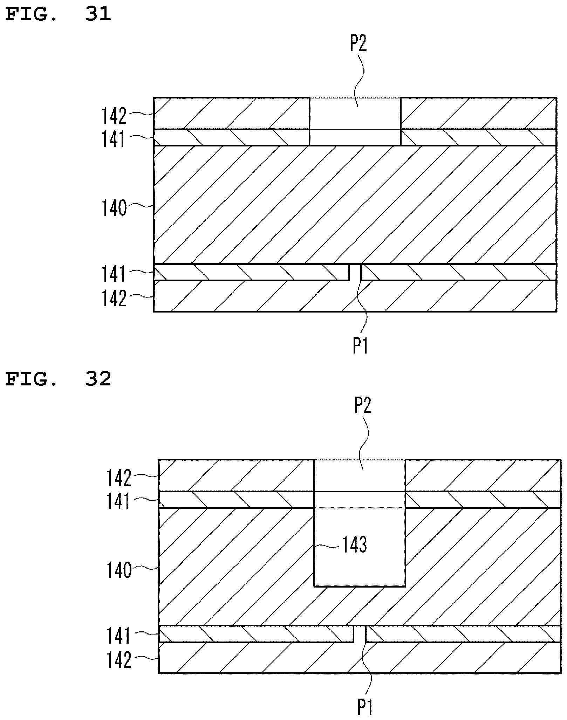

As illustrated in Fig. 31, in the back surface pattern forming step, the back surface pattern P2 is formed in the insulating film 141 and the protective film 142 provided on the back surface of the substrate 140. In the back surface pattern forming step, the substrate 140 is inverted and the back surface pattern P2 is formed at the position that corresponds to the surface pattern P1 in the insulating film 141 and the protective film 142 provided on the back surface of the substrate 140. The specific method for forming the back surface pattern P2 is similar to that in each of the embodiments described above, and thus description thereof is omitted. The insulating film 141 and the protective film 142 where the back surface pattern P2 is formed open at the part that corresponds to the back surface opening portion 136.

The upstream flow path 132 as an intra-substrate flow path is formed in the substrate 140 in the intra-substrate flow path forming step. The intra-substrate flow path forming step includes a first etching step, an inner wall protective film forming step, and a second etching step.

As illustrated in Fig. 32, in the first etching step, a hole 143 is formed by dry etching being performed on the back surface of the substrate 140. The hole 143 is formed so as to be perpendicular to the back surface of the substrate 140. The hole 143 has a side portion forming the second inner wall 132d. In this example, the dry etching in the first etching step is performed continuously with the dry etching in the back surface pattern forming step. Accordingly, the thickness of the photoresist layer that is used as a mask in the back surface pattern forming step is a thickness taking into account the depth of the hole 143 formed in the first etching step. In the first etching step, the dry etching is performed such that the substrate 140 is not penetrated. The remaining film thickness of the substrate 140 excluding the depth of the hole 143 is set based on the size of the bottom portion of the hole 143 and the size of the bottom portion of a hole 146 (described later) formed in the second etching step. For example, the film thickness is 120 µm in a case where the planar shape of the bottom portion of the hole 143 is a square in which the length of one side is 200 µm and the planar shape of the bottom portion of the hole 146 is a square in which the length of one side is 40 µm.

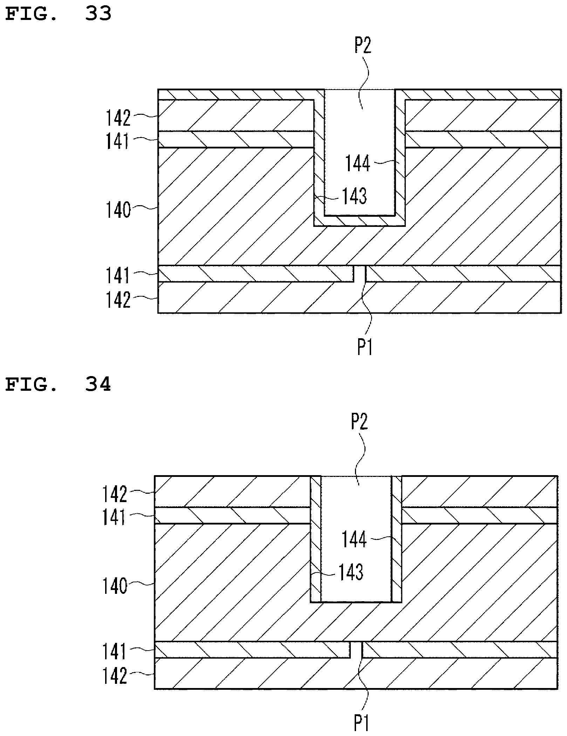

As illustrated in Fig. 33, an inner wall protective film 144 is formed in the hole 143 in the inner wall protective film forming step. In the inner wall protective film forming step, the inner wall protective film 144 is formed on the entire back surface of the substrate 140 first. As a result, the inner wall protective film 144 is formed in the hole 143, that is, on the bottom and side portions of the hole 143. The material of the inner wall protective film 144 is not particularly limited insofar as the material is selectively removable with respect to the substrate 140 and the insulating film 141. The inner wall protective film 144 is a SiO film in this example. The inner wall protective film 144 may be a metal film. The inner wall protective film 144 may be formed by a physical vapor deposition (PVD) method, such as a sputtering method, as well as a CVD method.

As illustrated in Fig. 34, in the subsequent inner wall protective film forming step, the part of the inner wall protective film 144 that is formed on the back surface of the substrate 140 and the part of the inner wall protective film 144 that is formed on the bottom portion of the hole 143 are removed by the inner wall protective film 144 being etched back. The inner wall protective film 144 remains on the side portion of the hole 143. A part of the substrate 140 is exposed from the bottom portion of the hole 143 by the inner wall protective film 144 on the bottom portion of the hole 143 being removed. It should be noted that how to form the inner wall protective film 144 is not limited to the method described above. For example, the substrate 140 may be heated in an oxygen atmosphere, the inner wall protective film 144 may be formed on the inner surface of the hole 143 by thermal oxidation of silicon, and the inner wall protective film 144 formed on the bottom portion of the hole 143 may be removed by being etched back. In a case where the inner wall protective film 144 is formed by thermal oxidation of silicon, it is preferable that the protective film 142 on the back surface of the substrate 140 is made thick in advance in view of how much is to be removed by the inner wall protective film 144 being etched back.

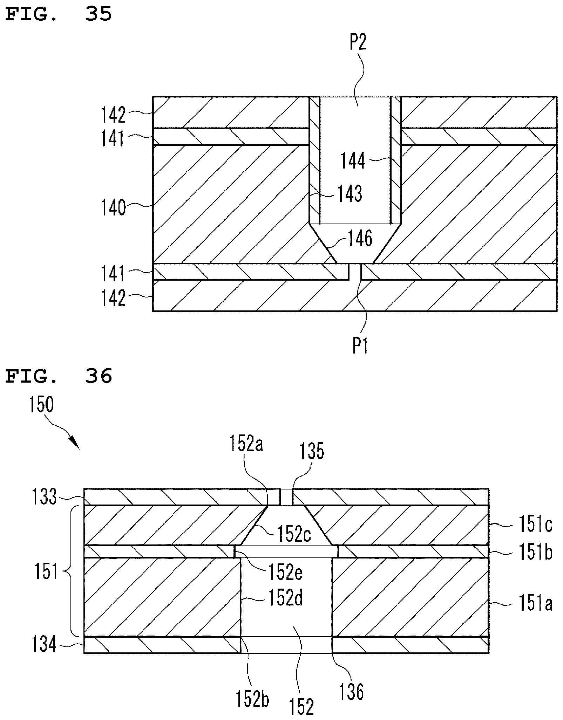

As illustrated in Fig. 35, in the second etching step, the hole 146 is formed in the substrate 140 by anisotropic wet etching being performed with the substrate 140 immersed in a wet etching solution. Used as the wet etching solution is an alkaline aqueous solution such as potassium hydroxide (KOH) and tetramethylammonium hydroxide (TMAH). The protective film 142 provided on both surfaces of the substrate 140 and the inner wall protective film 144 provided on the side portion of the hole 143 function as masks for anisotropic wet etching. Accordingly, the part of the back surface of the substrate 140 that is exposed from the bottom portion of the hole 143 is etched. The hole 146 is formed at the exposed part of the substrate 140 as a result of the anisotropic wet etching. The side portion of the hole 146 is inclined with respect to the back surface of the substrate 140. In this example, the side portion has an inclination angle of approximately 55°. The side portion of the hole 146 forms the first inner wall 132c. In the second etching step, the insulating film 141 provided on the surface of the substrate 140 is exposed from the bottom portion of the hole 146. The protective film 142 is formed on the entire surface of the substrate 140. Accordingly, damage to the surface is suppressed and etching from the surface is prevented during the anisotropic wet etching. After the anisotropic wet etching, the protective film 142 and the inner wall protective film 144 are removed by, for example, wet etching using HF as a wet etching solution. The fluid chip 130 illustrated in Fig. 29 is obtained as a result.

In the fluid chip 130, the first inner wall 132c is inclined with respect to the back surface of the substrate 131 and the second inner wall 132d is formed so as to be perpendicular to the back surface of the substrate 131. As a result, it is possible to reduce the difference between the opening area of the surface opening 132a and the opening area of the back surface opening 132b, and thus the fluid chip 130 itself can be reduced in size and the analysis device 41 can be reduced in size.

In the fluid chip 130, it is possible to provide a plurality of intra-substrate flow paths in the single substrate 131 by reducing the difference between the opening area of the surface opening 132a and the opening area of the back surface opening 132b. Accordingly, a plurality of samples can be efficiently analyzed.

In the fluid chip 130, the substrate 140 is etched by the dry etching in the first etching step and the anisotropic wet etching in the second etching step. As a result, the opening area of the surface opening 132a can be adjusted by the etching amount of the dry etching and the fluid chip 130 is excellent in terms of design freedom.

It should be noted that the fluid chip 130 is not limited to including the surface-side insulating film 133 and the back surface-side insulating film 134. The fluid chip 130 may include at least the surface-side insulating film 133.

### [Seventh Embodiment]

A silicon on insulator (SOI) substrate is used in a seventh embodiment whereas a silicon substrate is used as the substrate 131 in the sixth embodiment.

As illustrated in Fig. 36, a fluid chip 150 includes an SOI substrate 151 as a substrate, an upstream flow path 152 as an intra-substrate flow path, the surface-side insulating film 133, the back surface-side insulating film 134, the inflow opening portion 135, and the back surface opening portion 136. The surface-side insulating film 133, the back surface-side insulating film 134, the inflow opening portion 135, and the back surface opening portion 136 are identical to those in the sixth embodiment described above, and thus description thereof is omitted.

The SOI substrate 151 has a thickness of, for example, 775 µm. The SOI substrate 151 has a base substrate 151a, an insulating layer 151b, and a Si layer 151c. The base substrate 151a is, for example, a single crystal silicon substrate. The base substrate 151a has a thickness of approximately 670 µm. The insulating layer 151b is provided on the surface of the base substrate 151a. The insulating layer 151b is, for example, a SiO film. The thickness of the insulating layer 151b is approximately 2 µm. The Si layer 151c is provided on the surface of the insulating layer 151b. The thickness of the Si layer 151c is approximately 100 µm. The plane orientation of the Si layer 151c is, for example, (100) . It should be noted that a single crystal silicon substrate having a plane orientation of (100) as in the case of the Si layer 151c, a single crystal silicon substrate different in plane orientation from the Si layer 151c, or a polycrystalline silicon substrate can be used as the base substrate 151a.

The upstream flow path 152 is provided in the SOI substrate 151. The upstream flow path 152 penetrates the SOI substrate 151 in the thickness direction. The upstream flow path 152 has a surface opening 152a, a back surface opening 152b, a first inner wall 152c, a second inner wall 152d, and a third inner wall 152e.

The surface opening 152a is provided in the surface of the SOI substrate 151. The planar shape of the surface opening 152a is a circle or a polygon. In the present embodiment, the planar shape of the surface opening 152a is a square in which the length of one side is 40 µm.

The back surface opening 152b is provided in the back surface of the SOI substrate 151. The planar shape of the back surface opening 152b is a circle or a polygon. In the present embodiment, the planar shape of the back surface opening 152b is a square in which the length of one side is 200 µm.

The first inner wall 152c is provided in the Si layer 151c. The upper end of the first inner wall 152c is connected to the surface opening 152a. The lower end of the first inner wall 152c is connected to the upper end of the third inner wall 152e (described later) . The first inner wall 152c is inclined with respect to the back surface of the SOI substrate 151. The inclination angle of the first inner wall 152c with respect to the back surface of the SOI substrate 151 is approximately 55°.

The second inner wall 152d is provided in the base substrate 151a. In other words, the second inner wall 152d is provided downstream of the first inner wall 152c. The upper end of the second inner wall 152d is connected to the lower end of the third inner wall 152e (described later) . The lower end of the second inner wall 152d is connected to the back surface opening 152b. The second inner wall 152d is formed so as to be perpendicular to the back surface of the SOI substrate 151.

The third inner wall 152e is provided in the insulating layer 151b. In other words, the third inner wall 152e is provided between the first inner wall 152c and the second inner wall 152d. The upper end of the third inner wall 152e is connected to the lower end of the first inner wall 152c. The lower end of the third inner wall 152e is connected to the upper end of the second inner wall 152d. The third inner wall 152e is formed so as to be substantially perpendicular to the back surface of the SOI substrate 151. The third inner wall 152e is lower by one step than the wall surface of the second inner wall 152d.

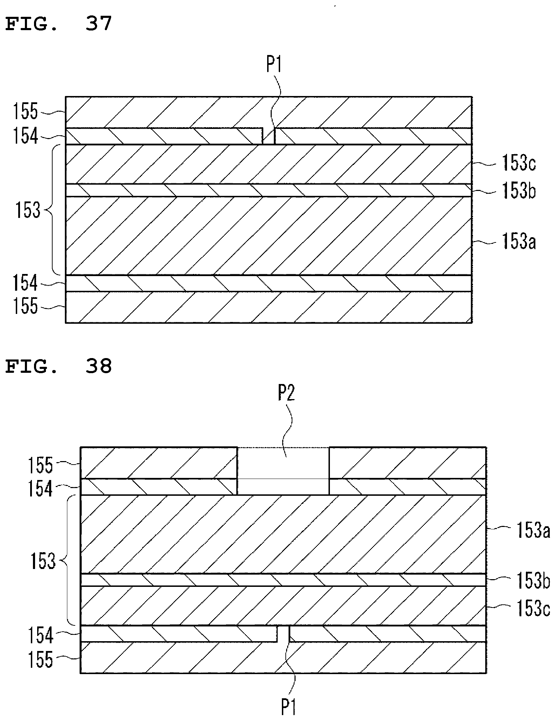

Hereinafter, a method for manufacturing the fluid chip 150 will be described with reference to Figs. 37 to 41. The fluid chip 130 is manufactured by a surface pattern forming step, a back surface pattern forming step, and an intra-substrate flow path forming step.

As illustrated in Fig. 37, in the surface pattern forming step, the surface pattern P1 is formed in the insulating film 141 provided on the surface of a SOI substrate 153. In the surface pattern forming step, the SOI substrate 153 is prepared first. In the SOI substrate 153, an insulating layer 153b made of a SiO film and a Si layer 153c are sequentially formed on the surface of a base substrate 153a. A single crystal silicon substrate, a polycrystalline silicon substrate, or the like is used as the base substrate 153a. The base substrate 153a is a single crystal silicon substrate in this example. Next, an insulating film 154 made of a SiN film is formed on both surfaces of the SOI substrate 153. Subsequently, the surface pattern P1 in which a part corresponding to the inflow opening portion 135 opens is formed in the insulating film 154 provided on the surface of the SOI substrate 153. The specific method for forming the surface pattern P1 is similar to that in each of the embodiments described above, and thus description thereof is omitted. In the surface pattern forming step, a protective film 155 made of a SiO film is formed on both surfaces of the SOI substrate 153 after the surface pattern P1 is formed.

As illustrated in Fig. 38, in the back surface pattern forming step, the back surface pattern P2 is formed in the insulating film 154 and the protective film 155 provided on the back surface of the SOI substrate 153. In the back surface pattern forming step, the SOI substrate 153 is inverted and the back surface pattern P2 is formed at the position that corresponds to the surface pattern P1 in the insulating film 154 and the protective film 155 provided on the back surface of the SOI substrate 153. The specific method for forming the back surface pattern P2 is similar to that in each of the embodiments described above, and thus description thereof is omitted. The insulating film 154 and the protective film 155 where the back surface pattern P2 is formed open at the part that corresponds to the back surface opening portion 136.

The upstream flow path 152 as an intra-substrate flow path is formed in the SOI substrate 153 in the intra-substrate flow path forming step. The intra-substrate flow path forming step includes a first etching step, an inner wall protective film forming step, and a second etching step.

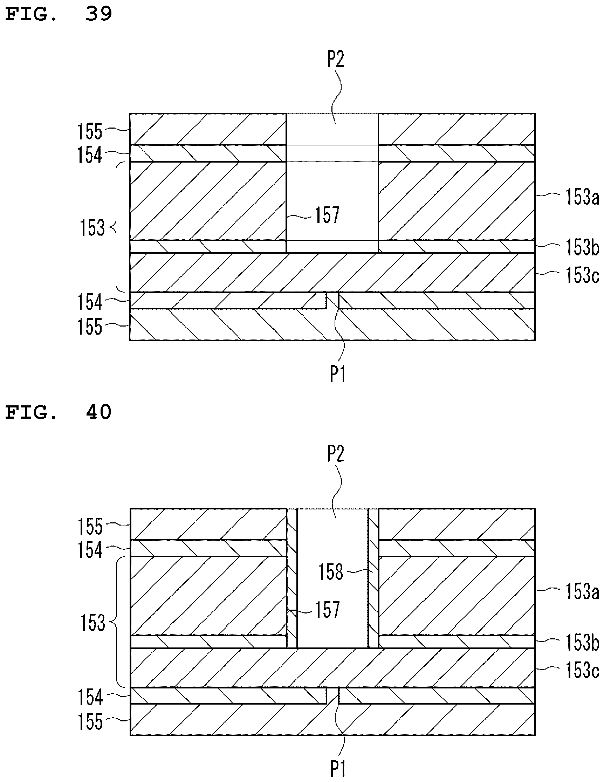

As illustrated in Fig. 39, in the first etching step, a hole 157 is formed by dry etching being performed on the back surface of the SOI substrate 153. The dry etching is performed until the base substrate 153a and the insulating layer 153b are penetrated and the Si layer 153c is reached. In other words, in the first etching step, the base substrate 153a is dry-etched first by the insulating layer 153b being used as an etching stopper, and then the insulating layer 153b is dry-etched by the Si layer 153c being used as an etching stopper. The hole 157 is formed so as to be perpendicular to the back surface of the SOI substrate 153. In the side portion of the hole 157, the part of the base substrate 153a forms the second inner wall 152d and the part of the insulating layer 153b forms the third inner wall 152e. In this example, the dry etching in the first etching step is performed continuously with the dry etching in the back surface pattern forming step. Accordingly, the thickness of the photoresist layer that is used as a mask in the back surface pattern forming step is a thickness taking into account the depth of the hole 157 formed in the first etching step. The remaining film thickness of the SOI substrate 153 excluding the depth of the hole 157 is set based on the size of the bottom portion of the hole 157 and the size of the bottom portion of a hole 159 (described later) formed in the second etching step.

As illustrated in Fig. 40, an inner wall protective film 158 is formed in the hole 157 in the inner wall protective film forming step. In the inner wall protective film forming step, the inner wall protective film 158 is formed on the entire back surface of the SOI substrate 153 first. Subsequently, the inner wall protective film 158 is etched back. As a result, the inner wall protective film 158 that is on the back surface of the SOI substrate 153 and the bottom portion of the hole 157 is removed. The inner wall protective film 158 remains on the side portion of the hole 157. A part of the Si layer 153c is exposed from the bottom portion of the hole 157 by the inner wall protective film 158 on the bottom portion of the hole 157 being removed.

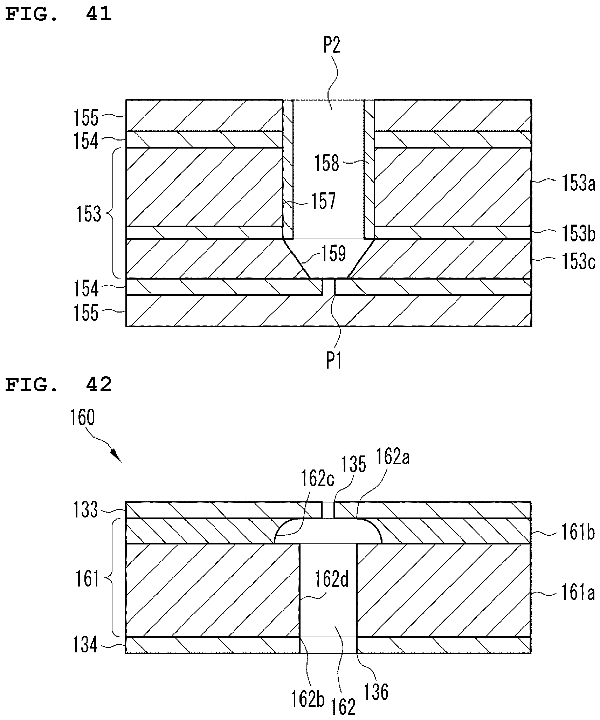

As illustrated in Fig. 41, in the second etching step, the hole 159 is formed in the SOI substrate 153 by anisotropic wet etching being performed with the SOI substrate 153 immersed in a wet etching solution. The inner wall protective film 158 and the protective film 155 provided on both surfaces of the SOI substrate 153 function as masks for anisotropic wet etching. As a result of the anisotropic wet etching, the hole 159 is formed at the part of the Si layer 153c that is exposed from the bottom portion of the hole 157. The side portion of the hole 159 is inclined with respect to the back surface of the SOI substrate 153. In this example, the side portion has an inclination angle of approximately 55°. The side portion of the hole 159 forms the first inner wall 152c. The insulating film 154 provided on the surface of the SOI substrate 153 is exposed from the bottom portion of the hole 159. After the anisotropic wet etching, the protective film 155 and the inner wall protective film 158 are removed by, for example, wet etching using HF as a wet etching solution. During the wet etching in this example, a part of the insulating layer 153b is removed and the third inner wall 152e becomes lower by one step than the second inner wall 152d. The fluid chip 150 illustrated in Fig. 36 is obtained as a result.

In the fluid chip 150, the first inner wall 152c is inclined with respect to the back surface of the SOI substrate 151 and the second inner wall 152d and the third inner wall 152e are formed so as to be perpendicular to the back surface of the SOI substrate 151. As a result, it is possible to reduce the difference between the opening area of the surface opening 152a and the opening area of the back surface opening 152b, and thus the fluid chip 150 itself can be reduced in size and the analysis device 41 can be reduced in size as in the case of the fluid chip 130. In addition, in the fluid chip 150, it is possible to provide a plurality of intra-substrate flow paths in the single SOI substrate 151, and thus a plurality of samples can be efficiently analyzed.

In the fluid chip 150, a single crystal silicon substrate or a polycrystalline silicon substrate can be used as the base substrate 153a in which the hole 157 is formed by the dry etching in the first etching step. The fluid chip 150 that is inexpensive can be obtained in a case where a polycrystalline silicon substrate is used as the base substrate 153a.

In the first etching step, dry etching is performed as the etching of the base substrate 153a of the SOI substrate 153. In the second etching step, anisotropic wet etching is performed as the etching of the Si layer 153c. Accordingly, it is possible to adjust the opening area of the surface opening 152a by changing the thickness of the Si layer 153c, and thus the fluid chip 150 is excellent in terms of design freedom.

In the first etching step, the base substrate 153a is dry-etched by the insulating layer 153b being used as an etching stopper. Accordingly, the opening area of the surface opening 152a can be adjusted more precisely than in the sixth embodiment.

It should be noted that the SOI substrate 151 may be replaced with a glass substrate on which a thin silicon substrate is affixed although the SOI substrate 151 has been described as an example in the seventh embodiment. In this case, the inner wall protective film forming step of forming the inner wall protective film 158 can be omitted in the intra-substrate flow path forming step.

### [Eighth Embodiment]

In the sixth embodiment, the first inner wall 132c is inclined at a specific inclination angle with respect to the back surface of the substrate 131. In an eighth embodiment, a first inner wall is curved in a concave shape.

As illustrated in Fig. 42, a fluid chip 160 includes a substrate 161, an upstream flow path 162 as an intra-substrate flow path, the surface-side insulating film 133, the back surface-side insulating film 134, the inflow opening portion 135, and the back surface opening portion 136. The substrate 161 has a base substrate 161a and a SiO film 161b. The base substrate 161a is, for example, a single crystal silicon substrate having a plane orientation of (100) . The SiO film 161b is provided on the surface of the base substrate 161a. The thickness of the SiO film 161b is, for example, 2 µm. It should be noted that the base substrate 161a may be a polycrystalline silicon substrate or a single crystal silicon substrate having a plane orientation different from the plane orientation of (100).

The upstream flow path 162 is provided in the substrate 161. The upstream flow path 162 penetrates the substrate 161 in the thickness direction. The upstream flow path 162 has a surface opening 162a, a back surface opening 162b, a first inner wall 162c, and a second inner wall 162d.

The surface opening 162a is provided in the surface of the substrate 161. The planar shape of the surface opening 162a is, for example, a square in which the length of one side is 40 µm. The back surface opening 162b is provided in the back surface of the substrate 161. The planar shape of the back surface opening 162b is a square in which the length of one side is 100 µm.

The first inner wall 162c is provided in the SiO film 161b. The first inner wall 162c is curved in a concave shape. The upper end of the first inner wall 162c is connected to the surface opening 162a. The lower end of the first inner wall 162c is connected to the upper end of the second inner wall 162d (described later) . The lower end of the first inner wall 162c is retracted from the opening end of the upper end of the second inner wall 162d.

The second inner wall 162d is provided in the base substrate 161a. In other words, the second inner wall 162d is provided downstream of the first inner wall 162c. The upper end of the second inner wall 162d is connected to the lower end of the first inner wall 162c. The lower end of the second inner wall 162d is connected to the back surface opening 162b. The second inner wall 162d is formed so as to be perpendicular to the back surface of the substrate 161. The size of the opening of the second inner wall 162d is substantially equal to that of the back surface opening 162b.

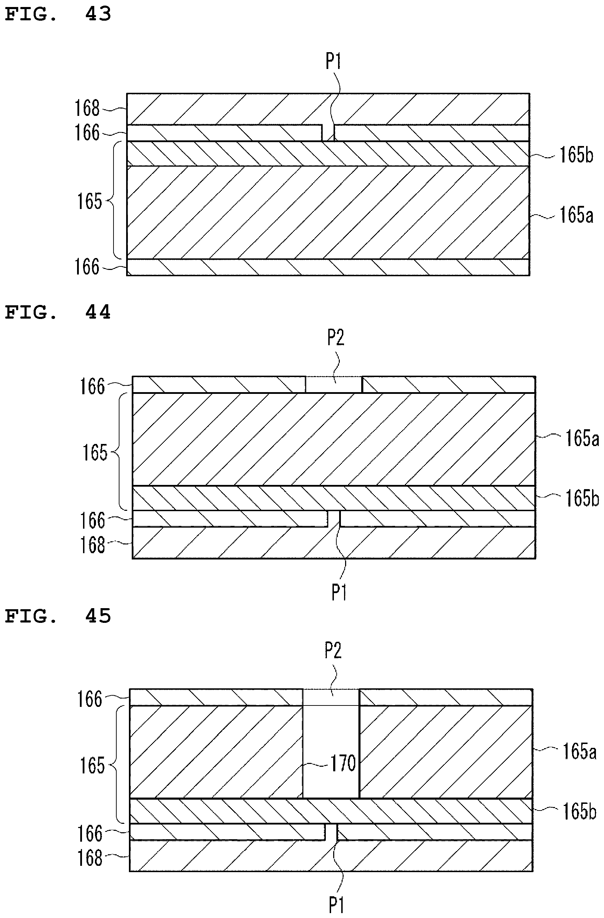

Hereinafter, a method for manufacturing the fluid chip 160 will be described with reference to Figs. 43 to 46. The fluid chip 130 is manufactured by a surface pattern forming step, a back surface pattern forming step, and an intra-substrate flow path forming step.

As illustrated in Fig. 43, in the surface pattern forming step, the surface pattern P1 is formed in an insulating film 166 provided on the surface of a substrate 165. In the surface pattern forming step, the substrate 165 is prepared first by a SiO film 165b being formed on the surface of a base substrate 165a. A single crystal silicon substrate, a polycrystalline silicon substrate, or the like is used as the base substrate 165a. The base substrate 165a is a single crystal silicon substrate in this example. The SiO film 167 is formed by, for example, a CVD method using TEOS as a source gas. Subsequently, the insulating film 166 is formed on both surfaces of the substrate 165. The insulating film 166 is formed by, for example, an LP-CVD method using DCS as a source gas. Next, the surface pattern P1 in which a part corresponding to the inflow opening portion 135 opens is formed in the insulating film 166 provided on the surface of the substrate 165. The specific method for forming the surface pattern P1 is similar to that in each of the embodiments described above, and thus description thereof is omitted. In the surface pattern forming step, a protective film 168 is formed on the surface of the substrate 165 after the surface pattern P1 is formed. The protective film 168 is set to a film thickness and a material that can be removed within the time required for the SiO film 165b to be etched during the isotropic wet etching performed in the second etching step to be described later.

As illustrated in Fig. 44, in the back surface pattern forming step, the back surface pattern P2 is formed in the insulating film 166 provided on the back surface of the substrate 165. In the back surface pattern forming step, the substrate 165 is inverted and the back surface pattern P2 is formed at the position that corresponds to the surface pattern P1 in the insulating film 166 provided on the back surface of the substrate 165. The specific method for forming the back surface pattern P2 is similar to that in each of the embodiments described above, and thus description thereof is omitted. The insulating film 166 where the back surface pattern P2 is formed opens at the part that corresponds to the back surface opening portion 136.

The upstream flow path 162 as an intra-substrate flow path is formed in the substrate 165 in the intra-substrate flow path forming step. The intra-substrate flow path forming step includes a first etching step and a second etching step.

As illustrated in Fig. 45, in the first etching step, a hole 170 is formed by dry etching being performed on the back surface of the substrate 165. In the first etching step, the base substrate 165a is dry-etched by the SiO film 165b being used as an etching stopper. The hole 170 is formed so as to be perpendicular to the back surface of the substrate 165. The hole 170 has a side portion forming the second inner wall 162d. In this example, the dry etching in the first etching step is performed continuously with the dry etching in the back surface pattern forming step.

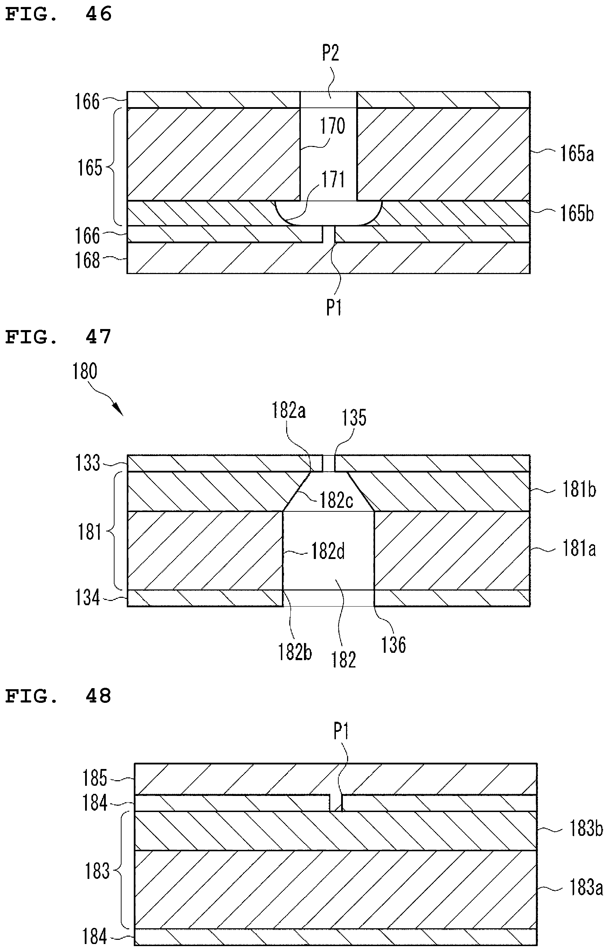

As illustrated in Fig. 46, in the second etching step, a hole 171 is formed in the SiO film 165b by isotropic wet etching being performed on the SiO film 165b. Wet etching using a wet etching solution such as HF is performed in the second etching step. The opening on the surface side of the substrate 165 is blocked by the protective film 168, and thus the wet etching solution flows into the hole 170 from the opening on the back surface side of the substrate 165. As a result of the wet etching, the hole 171 having a side portion curved in a concave shape is formed in the SiO film 165b. In the second etching step, the insulating film 166 provided on the surface of the substrate 165 is exposed from the bottom portion of the hole 171. The protective film 168 is also removed in the second etching step. The fluid chip 160 illustrated in Fig. 42 is obtained as a result.

In the fluid chip 160, it is possible to reduce the difference between the opening area of the surface opening 162a and the opening area of the back surface opening 162b as in the fluid chip 130 of the sixth embodiment, and thus the fluid chip 160 itself can be reduced in size and the analysis device 41 can be reduced in size. In addition, in the fluid chip 160, it is possible to provide a plurality of intra-substrate flow paths in the single substrate 161, and thus a plurality of samples can be efficiently analyzed.

In the fluid chip 160, a single crystal silicon substrate or a polycrystalline silicon substrate can be used as the base substrate 165a in which the hole 170 is formed by the dry etching in the first etching step. The fluid chip 160 that is inexpensive can be obtained in a case where a polycrystalline silicon substrate is used as the base substrate 165a.

In the first etching step, the base substrate 165a is dry-etched by the SiO film 165b being used as an etching stopper. Accordingly, the opening area of the surface opening 162a can be adjusted more precisely than in the sixth embodiment.

### [Ninth Embodiment]

As illustrated in Fig. 47, a fluid chip 180 includes an epi substrate 181 as a substrate, an upstream flow path 182 as an intra-substrate flow path, the surface-side insulating film 133, the back surface-side insulating film 134, the inflow opening portion 135, and the back surface opening portion 136.

The epi substrate 181 has a thickness of, for example, 775 µm. The epi substrate 181 has a base substrate 181a and an epi layer 181b. The base substrate 181a is, for example, a single crystal silicon substrate doped with P-type impurities . The impurity concentration of the base substrate 181a is 1E19/cm³ or more. The epi layer 181b is provided on the surface of the base substrate 181a. In this example, the epi layer 181b has a thickness of 100 µm and a volume resistivity of 10 Ω·cm. The impurity concentration of the epi layer 181b is lower than the impurity concentration of the base substrate 181a. The plane orientation of the epi layer 181b is, for example, (100). It should be noted that the base substrate 181a may be a single crystal silicon substrate having a plane orientation of (100) as in the case of the epi layer 181b, a single crystal silicon substrate different in plane orientation from the epi layer 181b, or a polycrystalline silicon substrate.

The upstream flow path 182 is provided in the epi substrate 181. The upstream flow path 182 penetrates the epi substrate 181 in the thickness direction. The upstream flow path 182 has a surface opening 182a, a back surface opening 182b, a first inner wall 182c, and a second inner wall 182d.

The surface opening 182a is provided in the surface of the epi substrate 181. The planar shape of the surface opening 182a is a circle or a polygon. The back surface opening 182b is provided in the back surface of the epi substrate 181. The planar shape of the back surface opening 182b is a circle or a polygon.

The first inner wall 182c is provided in the epi layer 181b. The upper end of the first inner wall 182c is connected to the surface opening 182a. The lower end of the first inner wall 182c is connected to the upper end of the second inner wall 182d. The first inner wall 182c is inclined with respect to the back surface of the epi substrate 181. The inclination angle of the first inner wall 182c is approximately 55°.

The second inner wall 182d is provided in the base substrate 181a. In other words, the second inner wall 182d is provided downstream of the first inner wall 182c. The upper end of the second inner wall 182d is connected to the lower end of the first inner wall 182c. The lower end of the second inner wall 182d is connected to the back surface opening 182b. The second inner wall 182d is formed so as to be perpendicular to the back surface of the epi substrate 181.

Hereinafter, a method for manufacturing the fluid chip 180 will be described with reference to Figs. 48 to 51. The fluid chip 180 is manufactured by a surface pattern forming step, a back surface pattern forming step, and an intra-substrate flow path forming step.

As illustrated in Fig. 48, in the surface pattern forming step, the surface pattern P1 is formed in an insulating film 184 provided on the surface of an epi substrate 183. In the surface pattern forming step, the epi substrate 183 is prepared first. In the epi substrate 183, an epi layer 183b is formed on the surface of a base substrate 183a. A single crystal silicon substrate, a polycrystalline silicon substrate, or the like is used as the base substrate 183a. The base substrate 183a is a single crystal silicon substrate in this example. Next, the insulating film 184 made of a SiN film is formed on both surfaces of the epi substrate 183. Subsequently, the surface pattern P1 in which a part corresponding to the inflow opening portion 135 opens is formed in the insulating film 184 provided on the surface of the epi substrate 183. The specific method for forming the surface pattern P1 is similar to that in each of the embodiments described above, and thus description thereof is omitted. In the surface pattern forming step, a protective film 185 made of a SiO film is formed on the surface of the epi substrate 183 after the surface pattern P1 is formed.

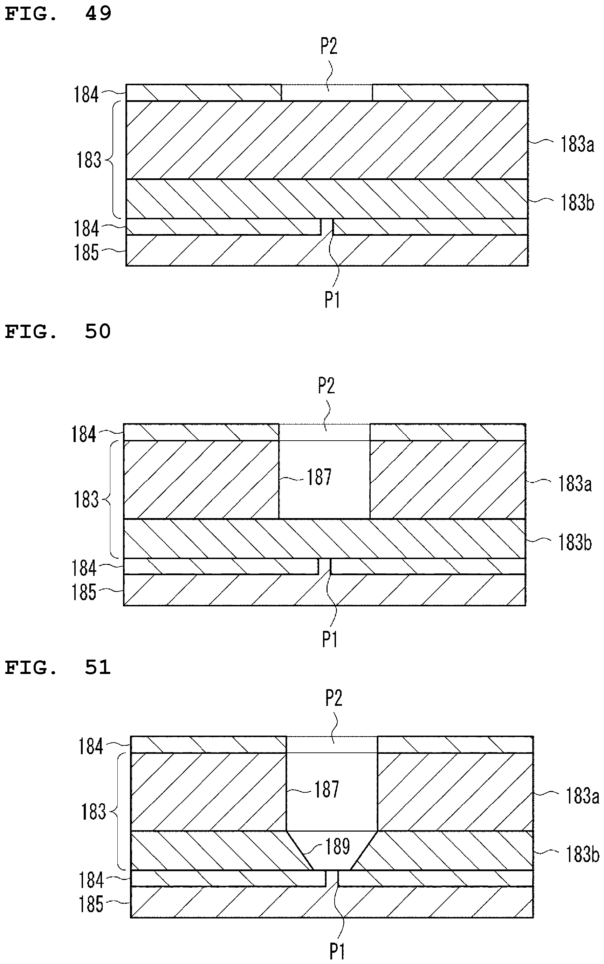

As illustrated in Fig. 49, in the back surface pattern forming step, the back surface pattern P2 is formed in the insulating film 184 provided on the back surface of the epi substrate 183. In the back surface pattern forming step, the epi substrate 183 is inverted and the back surface pattern P2 is formed at the position that corresponds to the surface pattern P1 in the insulating film 184 provided on the back surface of the epi substrate 183. The specific method for forming the back surface pattern P2 is similar to that in each of the embodiments described above, and thus description thereof is omitted. The insulating film 184 where the back surface pattern P2 is formed opens at the part that corresponds to the back surface opening portion 136.

The upstream flow path 182 as an intra-substrate flow path is formed in the epi substrate 183 in the intra-substrate flow path forming step. The intra-substrate flow path forming step includes a first etching step and a second etching step.

As illustrated in Fig. 50, in the first etching step, a hole 187 is formed by dry etching being performed on the back surface of the epi substrate 183. The dry etching is performed until the base substrate 183a is penetrated and the epi layer 183b is reached. In the first etching step, the epi layer 183b is exposed from the bottom portion of the hole 187. The hole 187 is formed so as to be perpendicular to the back surface of the epi substrate 183. The hole 187 has a side portion forming the second inner wall 182d. In this example, the dry etching in the first etching step is performed continuously with the dry etching in the back surface pattern forming step.

As illustrated in Fig. 51, in the second etching step, a hole 189 is formed in the epi substrate 183 by anisotropic wet etching being performed with the epi substrate 183 immersed in a wet etching solution. Used in the second etching step is a wet etching solution that is capable of selectively etching the epi layer 183b, which is lower in impurity concentration than the base substrate 183a of the epi substrate 183. An alkaline aqueous solution such as KOH and TMAH is used as the wet etching solution. As a result of the anisotropic wet etching, the hole 189 is formed at the part of the epi layer 183b that is exposed from the bottom portion of the hole 187. The side portion of the hole 189 is inclined with respect to the back surface of the epi substrate 183. In this example, the side portion has an inclination angle of approximately 55°. The side portion of the hole 189 forms the first inner wall 182c. In the second etching step, the insulating film 184 provided on the surface of the epi substrate 183 is exposed from the bottom portion of the hole 189. After the anisotropic wet etching, the protective film 185 is removed by, for example, wet etching using HF as a wet etching solution. The fluid chip 180 illustrated in Fig. 47 is obtained as a result.

In the fluid chip 180, it is possible to reduce the difference between the opening area of the surface opening 182a and the opening area of the back surface opening 182b as in the fluid chip 130 of the sixth embodiment, and thus the fluid chip 180 itself can be reduced in size and the analysis device 41 can be reduced in size. In addition, in the fluid chip 180, it is possible to provide a plurality of intra-substrate flow paths in the single epi substrate 181, and thus a plurality of samples can be efficiently analyzed.

In the fluid chip 180, a single crystal silicon substrate or a polycrystalline silicon substrate can be used as the base substrate 183a in which the hole 187 is formed by the dry etching in the first etching step. The fluid chip 180 that is inexpensive can be obtained in a case where a polycrystalline silicon substrate is used as the base substrate 183a.

In the first etching step, dry etching is performed as the etching of the base substrate 183a of the epi substrate 183. In the second etching step, anisotropic wet etching is performed as the etching of the epi layer 183b. Accordingly, it is possible to adjust the opening area of the surface opening 182a by changing the thickness of the epi layer 183b, and thus the fluid chip 180 is excellent in terms of design freedom.

It should be noted that an SOI substrate may be used instead of the epi substrate 181 that is used in the ninth embodiment described above. Used as the SOI substrate is one with a structure that has a base substrate having high-concentration impurities, an insulating layer, and a Si layer having a low impurity concentration.

The substrate of the fluid chip is capable of having a structure having a layer or a film where a first inner wall is formed by anisotropic wet etching on a base substrate where a second inner wall is formed by dry etching. Usable as the material of the base substrate where the second inner wall is formed and the material of the layer where the first inner wall is formed is a combination of materials different in etching rate with respect to a wet etching solution in anisotropic wet etching. The combination is, for example, a combination of N-type silicon and P-type silicon doped with impurities by ion implantation or a combination of N-type silicon and P-type silicon doped with impurities when a film is formed by a CVD method. In addition, one in which a silicon substrate and a compound semiconductor substrate are bonded together may be used as the substrate of the fluid chip.

### [Tenth Embodiment]

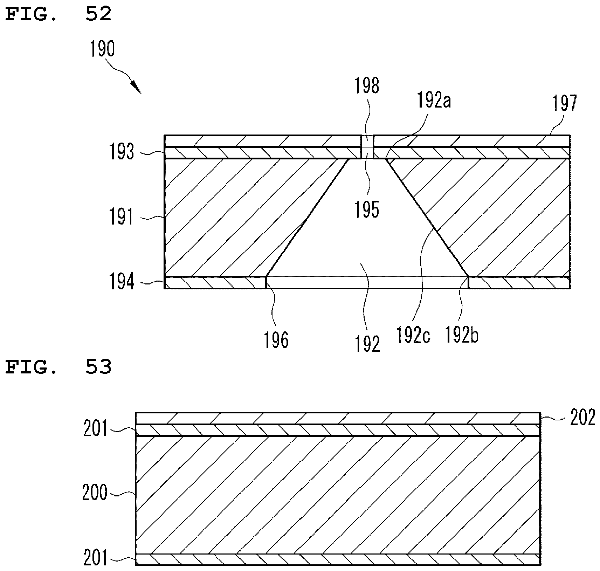

As illustrated in Fig. 52, a fluid chip 190 includes a substrate 191, an upstream flow path 192 as an intra-substrate flow path, a surface-side insulating film 193, a back surface-side insulating film 194, an inflow opening portion 195, a back surface opening portion 196, and a conductive film 197. The substrate 191 is, for example, a silicon substrate having a plane orientation of (100).

The upstream flow path 192 is provided in the substrate 191. The upstream flow path 192 penetrates the substrate 191 in the thickness direction. The upstream flow path 192 has a surface opening 192a, a back surface opening 192b, and an inner wall 192c.

The surface opening 192a opens the surface of the substrate 191. The surface opening 192a is connected to the inflow opening portion 195. The planar shape of the surface opening 192a is a circle, a polygon, or the like. In the present embodiment, the planar shape of the surface opening 192a is a square. The length of one side of the surface opening 192a is, for example, 40 µm.

The back surface opening 192b opens the back surface of the substrate 191. The back surface opening 192b is connected to the back surface opening portion 196. The planar shape of the back surface opening 192b is a circle, a polygon, or the like. In the present embodiment, the planar shape of the back surface opening 192b is a square. The length of one side of the back surface opening 192b is, for example, 1.1 mm.

The inner wall 192c is provided between the surface opening 192a and the back surface opening 192b. The upper end of the inner wall 192c is connected to the surface opening 192a. The lower end of the inner wall 192c is connected to the back surface opening 192b. The inner wall 192c is inclined with respect to the back surface of the substrate 191. The inclination angle of the inner wall 192c is approximately 55°.

The surface-side insulating film 193 is provided on the surface of the substrate 191. The surface-side insulating film 193 is formed of, for example, a SiN film or a SiO film. In this example, the surface-side insulating film 193 is formed of a SiN film. The thickness of the surface-side insulating film 193 is, for example, 20 nm.

The back surface-side insulating film 194 is provided on the back surface of the substrate 191. The back surface-side insulating film 194 is formed of, for example, a SiN film or a SiO film. In this example, the back surface-side insulating film 194 is formed of a SiN film as in the case of the surface-side insulating film 193. The thickness of the back surface-side insulating film 194 is, for example, 20 nm.

The inflow opening portion 195 is provided on the upstream side of the upstream flow path 192. The inflow opening portion 195 is formed in the surface-side insulating film 193 and is connected to the upstream flow path 192. The inflow opening portion 195 allows the sample to flow into the upstream flow path 192. The inflow opening portion 195 is the smallest opening portion in the sample flow channel in an analysis device 206 (described later) . The planar shape of the inflow opening portion 195 is a circle in the present embodiment. The diameter of the inflow opening portion 195 is, for example, 200 nm.

The back surface opening portion 196 is provided on the downstream side of the upstream flow path 192. The back surface opening portion 196 is formed in the back surface-side insulating film 194 and is connected to the upstream flow path 192. The back surface opening portion 196 allows the sample to flow out of the upstream flow path 192. The planar shape of the back surface opening portion 196 is a square in the present embodiment. The length of one side of the back surface opening portion 196 is, for example, 1.1 mm.

The conductive film 197 is provided in contact with the surface-side insulating film 193. The conductive film 197 is provided in contact with at least one of the surface and the back surface of the surface-side insulating film 193. In the present embodiment, the conductive film 197 is provided in contact with the surface of the surface-side insulating film 193. Although the conductive film 197 is provided on the entire surface of the surface-side insulating film 193 in the present embodiment, the conductive film 197 may be provided at least at a part corresponding to the inflow opening portion 195. The conductive film 197 is formed of a metal, a metal nitride film, or the like. Examples of the metal include titanium, tungsten, platinum, gold, cobalt, nickel, ruthenium, and tantalum. Examples of the metal nitride film include TiN and WN. The conductive film 197 may be formed of an alloy containing at least one type of metal selected from the above metals. In the present embodiment, a TiN film is used as the conductive film 197. The thickness of the conductive film 197 is, for example, 30 nm.

The conductive film 197 has a conductive film opening portion 198 connected to the inflow opening portion 195. The planar shape of the conductive film opening portion 198 is not particularly limited. In the present embodiment, the planar shape of the conductive film opening portion 198 is a circle as in the case of the inflow opening portion 195. The diameter of the conductive film opening portion 198 is set to a value equal to or larger than the diameter of the inflow opening portion 195. In the present embodiment, the diameter of the conductive film opening portion 198 is 200 nm as in the case of the inflow opening portion 195.

Hereinafter, a method for manufacturing the fluid chip 190 will be described with reference to Figs. 53 to 56. The fluid chip 190 is manufactured by a conductive film forming step, a surface pattern forming step, a back surface pattern forming step, and an intra-substrate flow path forming step.

As illustrated in Fig. 53, in the conductive film forming step, a conductive film 202 is formed on an insulating film 201 on the surface of a substrate 200 after the insulating film 201 is formed on both surfaces of the substrate 200. The substrate 200 is a silicon substrate. The thickness of the substrate 200 is 775 µm. The insulating film 201 is formed by, for example, a CVD method using DCS as a source gas. The conductive film 202 is formed by, for example, a reactive sputtering method using Ti as a target material and N₂ gas as an inert gas. As a result, the conductive film 202 is provided on the entire surface of the insulating film 201.

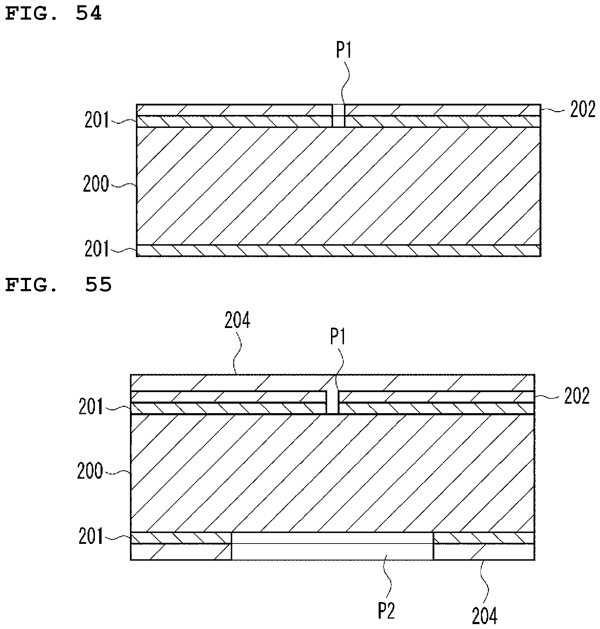

As illustrated in Fig. 54, in the surface pattern forming step, the surface pattern P1 is formed in the insulating film 201 and the conductive film 202 provided on the surface of the substrate 200. The surface pattern P1 is formed by, for example, a photoresist layer (not illustrated) being formed on the conductive film 202, the photoresist layer being patterned by the photolithography technique, and the conductive film 202 and the insulating film 201 on the surface of the substrate 200 being sequentially dry-etched by the patterned photoresist layer being used as a mask. The insulating film 201 where the surface pattern P1 is formed opens at the part that corresponds to the inflow opening portion 195. The conductive film 202 where the surface pattern P1 is formed opens at the part that corresponds to the conductive film opening portion 198.

As illustrated in Fig. 55, in the back surface pattern forming step, the back surface pattern P2 is formed in the insulating film 201 and a protective film 204 provided on the back surface of the substrate 200. In the back surface pattern forming step, the protective film 204 is formed on both surfaces of the substrate 200 first. The protective film 204 is preferably a material having a high etching rate selectivity with respect to a wet etching solution in anisotropic wet etching (described later). The protective film 32 is, for example, a SiO film formed by a CVD method using TEOS as a source gas. After the protective film 32 is formed, the back surface pattern P2 is formed in the insulating film 201 and the protective film 204 provided on the back surface of the substrate 200. The back surface pattern P2 and the surface pattern P1 can be formed by the same method, and thus the formation of the back surface pattern P2 will not be described. The insulating film 201 and the protective film 204 where the back surface pattern P2 is formed open at the part that corresponds to the back surface opening portion 196 and expose a part of the back surface of the substrate 200.

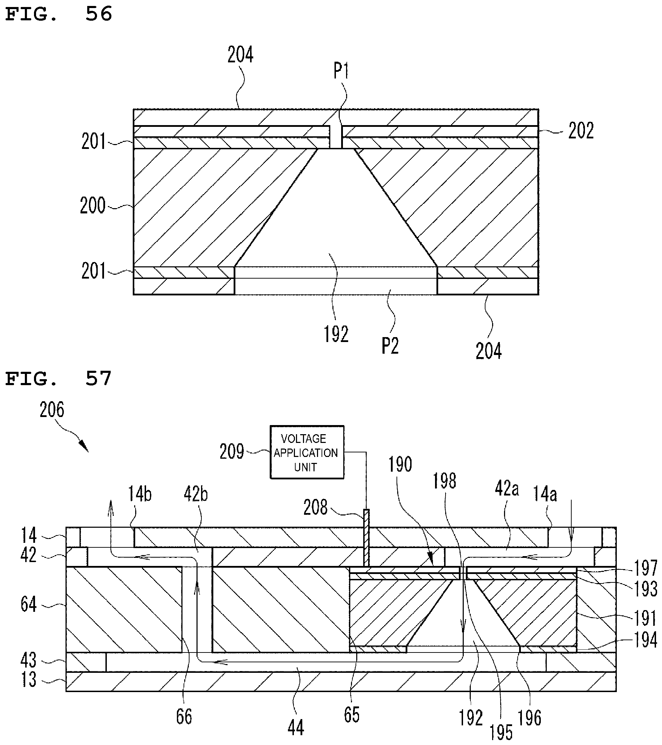

As illustrated in Fig. 56, in the intra-substrate flow path forming step, the upstream flow path 192 as an intra-substrate flow path is formed in the substrate 200. In the intra-substrate flow path forming step, anisotropic wet etching using, for example, an alkaline aqueous solution is performed. The protective film 204 functions as a mask for the anisotropic wet etching. The wet etching solution in the anisotropic wet etching enters the openings provided in the insulating film 201 and the protective film 204 by the back surface pattern P2 being formed. Etched as a result is a part of the substrate 200 exposed by the insulating film 201 and the protective film 204 where the back surface pattern P2 is formed. After the intra-substrate flow path forming step, the protective film 204 is removed by, for example, wet etching using HF. The fluid chip 190 illustrated in Fig. 52 is obtained as a result.

As described above, in the fluid chip 190, the conductive film 197 is provided in contact with the surface-side insulating film 193. Although the sample is analyzed by the change in ion current value at a time when the sample such as the DNA passes through the inflow opening portion 195 provided in the surface-side insulating film 193 being detected, static electricity may be generated and the sample may easily adhere by the surface-side insulating film being charged. In a case where the sample has adhered to the surface-side insulating film, the number of samples passing through the smallest opening portion with the smallest opening area in the sample flow channel decreases, it is impossible to measure the exact number of samples, and a decline in the precision of ion current measurement arises. In addition, in a case where the sample has adhered to the smallest opening portion, the smallest opening portion is blocked and the analysis of the sample becomes impossible. In the fluid chip 190, the conductive film 197 and the surface-side insulating film 193 are in contact with each other, and thus charging of the surface-side insulating film 193 is suppressed. Accordingly, in the fluid chip 190, sample adhesion to the surface-side insulating film 193 is prevented and the precision of ion current measurement can be maintained in a satisfactory manner. In addition, in the fluid chip 190, the inflow opening portion 195 is prevented from being blocked by the sample, and thus the sample can be analyzed in a reliable manner.

In the fluid chip 190, the surface-side insulating film 193 is reinforced by the conductive film 197, and thus the thickness of the surface-side insulating film 193 can be reduced. If the thickness of the surface-side insulating film is large, a plurality of samples will enter the inflow opening portion at the same time, a signal obtained by ion current measurement will become a signal based on the plurality of samples, and a decline in the precision of ion current measurement will arise. The smaller the thickness of the surface-side insulating film, the smaller the number of samples entering the inflow opening portion at the same time. As a result, the spatial resolution can be improved and the precision of ion current measurement can be improved. In the fluid chip 190, a high signal/noise ratio (S/N ratio) can be realized by the surface-side insulating film 193 being reduced in thickness.

In the tenth embodiment described above, a case where the conductive film 202 is continuously formed on the insulating film 201 on the surface of the substrate 200 in the conductive film forming step (see Fig. 53) has been described. Alternatively, in a case where the film thickness of the conductive film 197 is sufficiently smaller than the diameter of the inflow opening portion 195, a conductive film may be formed on the insulating film 201 on the surface of the substrate 200 after the protective film 204 (see Fig. 56) is removed through the surface pattern forming step, the back surface pattern forming step, and the intra-substrate flow path forming step without the continuous formation of the conductive film 202 on the insulating film 201 on the surface of the substrate 200. In other words, the conductive film forming step may be performed after the intra-substrate flow path forming step. In this case, the film thickness of the conductive film is less than 50% of the diameter of the inflow opening portion 195. As a result, the inflow opening portion 195 is prevented from being blocked when the conductive film is formed by a sputtering method or a CVD method. In a case where the conductive film forming step is performed after the intra-substrate flow path forming step, a thin conductive film is also formed inside the opening of the insulating film 201. As a result, it is possible to adjust the diameter of the inflow opening portion 195 by adjusting the film thickness of the conductive film formed on the insulating film 201.

Fig. 57 is a schematic cross-sectional view illustrating the analysis device 206 in which the fluid chip 190 is implemented. The analysis device 206 includes the lower-side cover sheet 13, the upper-side cover sheet 14, the upper-side flow path sheet 42, the lower-side flow path sheet 43, the chip frame 64, and so on in addition to the fluid chip 190. The fluid chip 190 is held by the chip frame 64. It should be noted that the analysis device 206 includes the electrode pair 15 (see Fig. 1), which is not illustrated in Fig. 57, and the electrode pair 15 is provided in the supply portion 14a and the collection portion 14b provided in the upper-side cover sheet 14. The analysis device 206 forms a flow channel for sample passage between the supply portion 14a and the collection portion 14b. The smallest opening portion of the flow channel is the inflow opening portion 195 of the fluid chip 190.

The analysis device 206 further includes a control electrode 208 provided on the conductive film 197 and a voltage application unit 209 applying a voltage to the control electrode 208. In Fig. 57, the control electrode 208 is formed in a rod shape and is disposed in a through hole (not illustrated) penetrating the upper-side cover sheet 14 and the upper-side flow path sheet 42. One end of the control electrode 208 is connected to the conductive film 197. The other end of the control electrode 208 protrudes from the upper surface of the upper-side cover sheet 14.

The voltage application unit 209 is electrically connected to the control electrode 208. In this example, the voltage application unit 209 is connected to the other end of the control electrode 208. The voltage application unit 209 controls the potential of the conductive film 197 by applying a positive or negative voltage to the control electrode 208.

As described above, the analysis device 206 includes the control electrode 208 and the voltage application unit 209 and is capable of controlling the potential of the conductive film 197. Accordingly, static elimination can be performed on the surface-side insulating film 193 via the conductive film 197 even in a case where the surface-side insulating film 193 is charged. "Static elimination" includes not only the post-static elimination charge amount of a static elimination object completely becoming zero but also the post-static elimination charge amount being smaller than the pre-static elimination charge amount. Accordingly, the analysis device 206 reliably prevents sample adhesion to the surface-side insulating film 193.

The analysis device 206 changes the polarity of the potential of the conductive film 197 in accordance with the polarity of the sample to be analyzed. As a result, sample adhesion to the surface-side insulating film 193 is prevented in a more reliable manner. For example, in a case where a positively charged sample is analyzed, the analysis device 206 sets the potential of the conductive film 197 to the positive polarity, which is the polarity of the sample, and electrically repels the sample and the surface-side insulating film 193. As a result, sample adhesion to the surface-side insulating film 193 is prevented in a more reliable manner.

### [Eleventh Embodiment]

In the tenth embodiment described above, the conductive film 197 is provided on the surface of the surface-side insulating film 193. In an eleventh embodiment, a conductive film is provided on the back surface of the surface-side insulating film.

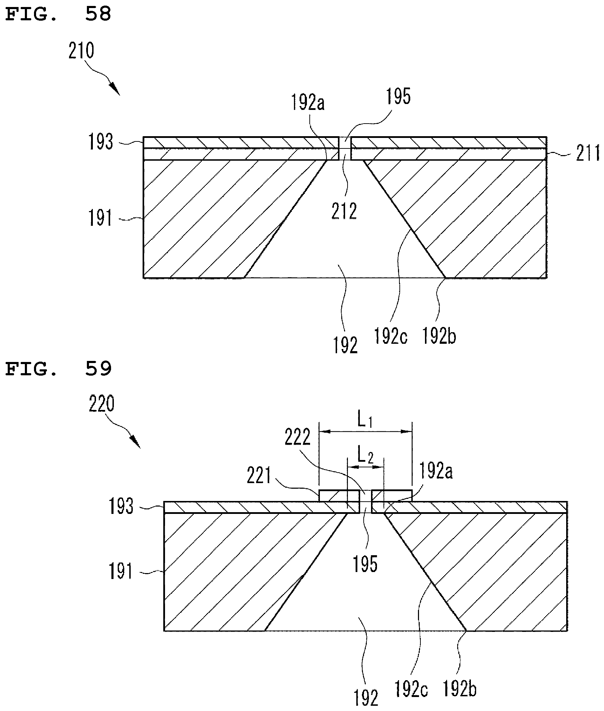

As illustrated in Fig. 58, a fluid chip 210 includes the substrate 191, the upstream flow path 192 as an intra-substrate flow path, the surface-side insulating film 193, the inflow opening portion 195, and a conductive film 211. It should be noted that the fluid chip 210 does not include the back surface-side insulating film 194 and the back surface opening portion 196 (see Fig. 52) whereas the fluid chip 190 of the tenth embodiment includes the back surface-side insulating film 194 and the back surface opening portion 196. The substrate 191, the upstream flow path 192, the surface-side insulating film 193, and the inflow opening portion 195 are identical to those in the tenth embodiment described above, and thus description thereof is omitted.

The conductive film 211 is provided in contact with the back surface of the surface-side insulating film 193. In other words, the conductive film 211 is disposed between the substrate 191 and the surface-side insulating film 193. The conductive film 211 is similar to the conductive film 197 except for the disposition. In other words, the conductive film 211 is formed of a metal, an alloy, a metal nitride film, or the like.

The conductive film 211 has a conductive film opening portion 212 connected to the inflow opening portion 195. The planar shape of the conductive film opening portion 212 is not particularly limited. The diameter of the conductive film opening portion 212 is set to a value equal to or larger than the diameter of the inflow opening portion 195. In the present embodiment, the conductive film opening portion 212 has a circular planar shape and a diameter of 200 nm.

The method for manufacturing the fluid chip 210 is the same as the method for manufacturing the fluid chip 190 except for the conductive film forming step. In the conductive film forming step as a method for manufacturing the fluid chip 210, a conductive film and an insulating film are formed in this order on the surface of the substrate and neither a conductive film nor an insulating film are formed on the back surface of the substrate. Description of the surface pattern forming step, the back surface pattern forming step, and the intra-substrate flow path forming step is omitted.

As described above, in the fluid chip 210, the conductive film 211 is provided on the back surface of the surface-side insulating film 193 and the conductive film 211 and the surface-side insulating film 193 are in contact with each other. As a result, charging of the surface-side insulating film 193 is suppressed. Accordingly, the fluid chip 210 is capable of maintaining the precision of ion current measurement in a satisfactory manner and is capable of reliably analyzing the sample as in the case of the fluid chip 190. In addition, in the fluid chip 210, the surface-side insulating film 193 is reinforced by the conductive film 211, the surface-side insulating film 193 can be reduced in thickness, and thus a high S/N ratio can be realized.

The fluid chip 210 can be used in the analysis device 206 (see Fig. 57) instead of the fluid chip 190. In the analysis device 206 that includes the fluid chip 210, for example, the control electrode 208 is disposed on the side surface of the fluid chip 210 and the control electrode 208 and the conductive film 211 of the fluid chip 210 are interconnected. The control electrode 208 may be provided so as to penetrate the surface-side insulating film 193 and be connected to the conductive film 211. As a result, in the analysis device 206 that includes the fluid chip 210, static elimination can be performed on the surface-side insulating film 193 via the conductive film 211, and thus sample adhesion to the surface-side insulating film 193 is reliably prevented. In addition, the analysis device 206 that includes the fluid chip 210 changes the polarity of the potential of the conductive film 211 in accordance with the polarity of the sample to be analyzed. As a result, sample adhesion to the surface-side insulating film 193 is prevented in a more reliable manner.

Although the conductive film 211 is provided on the back surface of the surface-side insulating film 193 in the eleventh embodiment, the conductive film 197 may be further provided on the surface of the surface-side insulating film 193 as in the tenth embodiment. In other words, conductive films may be provided on both surfaces of the surface-side insulating film.

### [Twelfth Embodiment]

In the tenth embodiment described above, the conductive film 197 is provided on the entire surface of the surface-side insulating film 193. In a twelfth embodiment, a conductive film is provided at a part of the surface-side insulating film.

As illustrated in Fig. 59, a fluid chip 220 includes the substrate 191, the upstream flow path 192 as an intra-substrate flow path, the surface-side insulating film 193, the inflow opening portion 195, and a conductive film 221.

The conductive film 221 is provided in contact with the surface of the surface-side insulating film 193. The conductive film 221 is provided at a part of the surface of the surface-side insulating film 193. More specifically, the conductive film 221 is provided at the part of the surface of the surface-side insulating film 193 that corresponds to the inflow opening portion 195. It should be noted that the conductive film 221 may be provided on the back surface of the surface-side insulating film 193 or on both surfaces of the surface-side insulating film 193 although the conductive film 221 is provided on the surface of the surface-side insulating film 193 in the present embodiment. The conductive film 221 is formed of a metal, an alloy, a metal nitride film, or the like.

In Fig. 59, L₁ is the size of the conductive film 221 and L₂ is the size of the surface opening 192a of the upstream flow path 192. In the present embodiment, the conductive film 221 has a square planar shape and the surface opening 192a has a square planar shape, and thus L₁ is the length of one side of the conductive film 221 and L₂ is the length of one side of the surface opening 192a. The length L₁ of one side of the conductive film 221 exceeds the length L₂ of one side of the surface opening 192a of the upstream flow path 192. In other words, the conductive film 221 is larger in size than the surface opening 192a. It should be noted that Li is the diameter of the conductive film 221 and L₂ is the diameter of the surface opening 192a in a case where, for example, the conductive film 221 has a circular planar shape and the surface opening 192a has a circular planar shape.

The conductive film 221 has a conductive film opening portion 222 connected to the inflow opening portion 195. The planar shape of the conductive film opening portion 222 is not particularly limited. The diameter of the conductive film opening portion 222 is set to a value equal to or larger than the diameter of the inflow opening portion 195. In the present embodiment, the conductive film opening portion 222 has a circular planar shape and a diameter of 200 nm.

The method for manufacturing the fluid chip 220 is the same as the method for manufacturing the fluid chip 190 except for the conductive film forming step. In the conductive film forming step as a method for manufacturing the fluid chip 220, a conductive film is dry-etched into a predetermined shape after an insulating film and the conductive film are formed in this order on the surface of the substrate. Description of the surface pattern forming step, the back surface pattern forming step, and the intra-substrate flow path forming step is omitted.

As described above, in the fluid chip 220, the conductive film 221 and the surface-side insulating film 193 are in contact with each other. As a result, charging of the surface-side insulating film 193 is suppressed. Accordingly, the fluid chip 220 is capable of maintaining the precision of ion current measurement in a satisfactory manner and is capable of reliably analyzing the sample as in the case of the fluid chip 190. In addition, in the fluid chip 220, the conductive film 221 reinforces the surface-side insulating film 193 that is in a region not supported by the substrate 191, the surface-side insulating film 193 can be reduced in thickness, and thus a high S/N ratio can be realized.

The fluid chip 220 can be used in the analysis device 206 (see Fig. 57) instead of the fluid chip 190. In the analysis device 206 that includes the fluid chip 220, static elimination can be performed on the surface-side insulating film 193 via the conductive film 221, and thus sample adhesion to the surface-side insulating film 193 is reliably prevented. In addition, the analysis device 206 that includes the fluid chip 220 changes the polarity of the potential of the conductive film 211 in accordance with the polarity of the sample to be analyzed. As a result, sample adhesion to the surface-side insulating film 193 is prevented in a more reliable manner.

### [Thirteenth Embodiment]

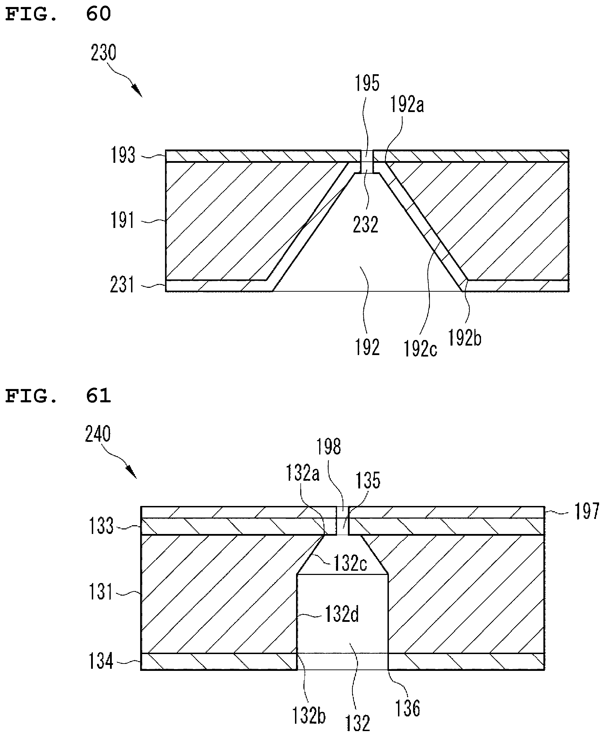

As illustrated in Fig. 60, a fluid chip 230 includes the substrate 191, the upstream flow path 192 as an intra-substrate flow path, the surface-side insulating film 193, the inflow opening portion 195, and a conductive film 231.

The conductive film 231 is provided in the upstream flow path 192 as an intra-substrate flow path. In Fig. 60, the conductive film 231 is provided in the upstream flow path 192 and on the back surface of the substrate 191. The conductive film 231 is not provided at the part of the upstream flow path 192 that corresponds to the inflow opening portion 195. The conductive film 231 is in contact with the back surface of the surface-side insulating film 193 that is exposed from the surface opening 192a of the upstream flow path 192. The conductive film 231 is formed of a metal, an alloy, a metal nitride film, or the like.

The conductive film 231 has a conductive film opening portion 232 connected to the inflow opening portion 195. The planar shape of the conductive film opening portion 232 is not particularly limited. The diameter of the conductive film opening portion 232 is set to a value equal to or larger than the diameter of the inflow opening portion 195. In the present embodiment, the conductive film opening portion 232 has a circular planar shape and a diameter of 200 nm.

The method for manufacturing the fluid chip 230 has a surface pattern forming step, a back surface pattern forming step, an intra-substrate flow path forming step, and a conductive film forming step. In the surface pattern forming step, an insulating film is formed on the surface of the substrate and a surface pattern is formed in the insulating film. With the surface pattern formed, the part of the insulating film that corresponds to the inflow opening portion 195 opens. In the back surface pattern forming step, a protective film is formed on both surfaces of the substrate and a back surface pattern is formed in the protective film that is on the back surface of the substrate. The protective film that is on the surface of the substrate blocks the opening that is formed in the insulating film. In the intra-substrate flow path forming step, the upstream flow path 192 as an intra-substrate flow path is formed in the substrate. In the conductive film forming step, the protective film provided on both surfaces of the substrate is removed first. After the protective film is removed, a conductive film is formed in the upstream flow path 192 by a sputtering method. By a highly directional anisotropic sputtering method being used, no conductive film is formed inside the opening of the insulating film, that is, at the part corresponding to the inflow opening portion 195. The conductive film is formed on the back surface of the substrate 191, the inclined inner wall 192c of the upstream flow path 192, and the back surface of the insulating film exposed from the surface opening 192a of the upstream flow path 192.

As described above, in the fluid chip 230, the conductive film 231 and the surface-side insulating film 193 are in contact with each other. As a result, charging of the surface-side insulating film 193 is suppressed. Accordingly, the fluid chip 230 is capable of maintaining the precision of ion current measurement in a satisfactory manner and is capable of reliably analyzing the sample as in the case of the fluid chip 190. In addition, in the fluid chip 230, the conductive film 231 reinforces the surface-side insulating film 193, the surface-side insulating film 193 can be reduced in thickness, and thus a high S/N ratio can be realized.

The fluid chip 230 can be used in the analysis device 206 (see Fig. 57) instead of the fluid chip 190. In the analysis device 206 that includes the fluid chip 230, for example, the control electrode 208 is disposed on the side surface of the fluid chip 230 and the control electrode 208 and the conductive film 231 of the fluid chip 230 are interconnected. In the analysis device 206 that includes the fluid chip 230, static elimination can be performed on the surface-side insulating film 193 via the conductive film 231, and thus sample adhesion to the surface-side insulating film 193 is reliably prevented. In addition, the analysis device 206 that includes the fluid chip 230 changes the polarity of the potential of the conductive film 231 in accordance with the polarity of the sample to be analyzed. As a result, sample adhesion to the surface-side insulating film 193 is prevented in a more reliable manner.

### [Fourteenth Embodiment]

In a fourteenth embodiment, the fluid chip 130 of the sixth embodiment is provided with the conductive film 197 of the tenth embodiment.

As illustrated in Fig. 61, a fluid chip 240 includes the substrate 131, the upstream flow path 132 as an intra-substrate flow path, the surface-side insulating film 133, the back surface-side insulating film 134, the inflow opening portion 135, the back surface opening portion 136, and the conductive film 197. The upstream flow path 132 has the surface opening 132a, the back surface opening 132b, the first inner wall 132c, and the second inner wall 132d. The conductive film 197 is provided on the surface of the surface-side insulating film 133 and is in contact with the surface-side insulating film 133. The conductive film opening portion 198 of the conductive film 197 is connected to the inflow opening portion 135.

In the fluid chip 240, the conductive film 197 and the surface-side insulating film 133 are in contact with each other, and thus charging of the surface-side insulating film 133 is suppressed. Accordingly, the fluid chip 240 is capable of maintaining the precision of ion current measurement in a satisfactory manner and is capable of reliably analyzing the sample. In addition, in the fluid chip 240, the conductive film 197 reinforces the surface-side insulating film 133, the surface-side insulating film 133 can be reduced in thickness, and thus a high S/N ratio can be realized. Further, the fluid chip 240 is capable of being similar in action and effect to the fluid chip 130 of the sixth embodiment.

### [Fifteenth Embodiment]

In a fifteenth embodiment, the fluid chip 160 of the eighth embodiment is provided with the conductive film 197 of the tenth embodiment.

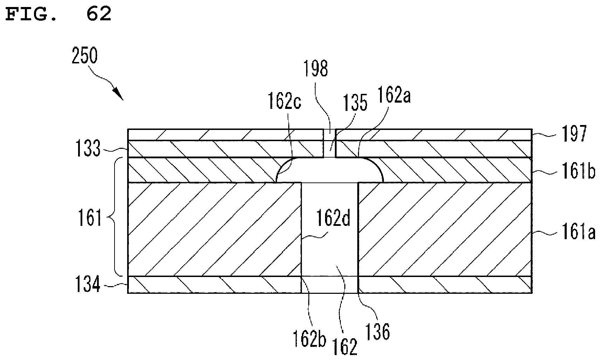

As illustrated in Fig. 62, a fluid chip 250 includes the substrate 161, the upstream flow path 162 as an intra-substrate flow path, the surface-side insulating film 133, the back surface-side insulating film 134, the inflow opening portion 135, the back surface opening portion 136, and the conductive film 197. The substrate 161 has the base substrate 161a and the SiO film 161b. The upstream flow path 162 has the surface opening 162a, the back surface opening 162b, the first inner wall 162c, and the second inner wall 162d. The conductive film 197 is provided on the surface of the surface-side insulating film 133 and is in contact with the surface-side insulating film 133. The conductive film opening portion 198 of the conductive film 197 is connected to the inflow opening portion 135.

In the fluid chip 250, the conductive film 197 and the surface-side insulating film 133 are in contact with each other, and thus charging of the surface-side insulating film 133 is suppressed. Accordingly, the fluid chip 250 is capable of maintaining the precision of ion current measurement in a satisfactory manner and is capable of reliably analyzing the sample. In addition, in the fluid chip 250, the conductive film 197 reinforces the surface-side insulating film 133, the surface-side insulating film 133 can be reduced in thickness, and thus a high S/N ratio can be realized. Further, the fluid chip 250 is capable of being similar in action and effect to the fluid chip 160 of the eighth embodiment.

The invention is not limited to the above-described embodiments as they are and can be embodied with constituent elements modified within the scope of the gist of the invention in an implementation stage. In addition, various inventions can be formed by the plurality of constituent elements disclosed in the embodiments being appropriately combined. Also conceivable is, for example, a configuration that lacks some of the constituent elements illustrated in the embodiments . Further, the constituent elements described in the different embodiments may be combined as appropriate.

### Reference Signs List

10, 40, 60, 70, 90, 130, 150, 160, 180, 190, 210, 220, 230, 240, 250 Fluid chip
11, 41, 120, 206 Analysis device
12, 42, 122 Upper-side flow path sheet
12a, 42a, 122a First upper-side flow path
12b, 42b, 42c, 122b Second upper-side flow path
14a, 14d Supply portion
14b, 14c Collection portion
15 Electrode pair
16, 64, 124 Chip frame
21, 30, 53, 63, 80, 131, 140, 151, 161, 191 Substrate
22, 61, 133, 193 Surface-side insulating film
22a, 92a, 102, 135, 195 Inflow opening portion
22b, 92b Outflow opening portion
23, 52, 62, 134, 194 Back surface-side insulating film
23a Upstream-side back surface opening portion
23b Downstream-side back surface opening portion
26, 132, 152, 162, 182, 192 Upstream flow path
27 Downstream flow path
28 Back surface flow path
43, 123 Lower-side flow path sheet
44 Lower-side flow path
74 Reinforcing film
54, 66 Connection hole
123a First lower-side flow path
123b Second lower-side flow path
136, 196 Back surface opening portion
197, 211, 221, 231 Conductive film
198, 212, 222, 232 Conductive film opening portion

## Claims

1. A fluid chip comprising:
an intra-substrate flow path provided in a substrate;
an insulating film provided on a surface of the substrate;
an inflow opening portion provided on an upstream side of the intra-substrate flow path and allowing a sample to flow into the intra-substrate flow path; and
an outflow opening portion provided on a downstream side of the intra-substrate flow path and allowing the sample to flow out of the intra-substrate flow path,
wherein the inflow opening portion and the outflow opening portion are provided in the insulating film and interconnected via the intra-substrate flow path.

2. The fluid chip according to claim 1, wherein the intra-substrate flow path has:
an upstream flow path connected to the inflow opening portion;
a downstream flow path connected to the outflow opening portion; and
a back surface flow path provided in a back surface of the substrate and interconnecting the upstream flow path and the downstream flow path.

3. The fluid chip according to claim 1 or 2, further comprising a reinforcing film provided between the substrate and the insulating film.

4. An analysis device comprising:
the fluid chip according to claim 2;
an upper-side sheet provided on a surface of the fluid chip;
a supply portion where the sample is supplied; and
a collection portion where the sample is collected, wherein
the upper-side sheet has a first upper-side flow path interconnecting the supply portion and the inflow opening portion and a second upper-side flow path interconnecting the collection portion and the outflow opening portion, and
a flow channel allowing the sample to flow between the supply portion and the collection portion is formed.

5. An analysis device comprising:
the fluid chip according to claim 1;
an upper-side sheet provided on a surface of the fluid chip;
a lower-side sheet provided on a back surface of the fluid chip;
a supply portion where the sample is supplied; and
a collection portion where the sample is collected, wherein
the upper-side sheet has a first upper-side flow path interconnecting the supply portion and the inflow opening portion and a second upper-side flow path interconnecting the collection portion and the outflow opening portion,
the intra-substrate flow path has an upstream flow path connected to the inflow opening portion and a downstream flow path connected to the outflow opening portion,
the lower-side sheet has a lower-side flow path interconnecting the upstream flow path and the downstream flow path, and
a flow channel allowing the sample to flow between the supply portion and the collection portion is formed.

6. An analysis device comprising:
a fluid chip having an intra-substrate flow path penetrating a substrate having a surface on which an insulating film is provided;
an upper-side sheet provided on a surface of the fluid chip;
a lower-side sheet provided on a back surface of the fluid chip;
a chip frame provided between the upper-side sheet and the lower-side sheet and holding the fluid chip;
a supply portion where a sample is supplied; and
a collection portion where the sample is collected, wherein
the upper-side sheet has a first upper-side flow path connected to the supply portion and a second upper-side flow path connected to the collection portion,
the chip frame has a connection hole connected to the second upper-side flow path,
the insulating film has an inflow opening portion connected to the first upper-side flow path and allowing the sample to flow into the intra-substrate flow path,
the lower-side sheet has a lower-side flow path interconnecting the intra-substrate flow path and the connection hole, and
a flow channel allowing the sample to flow between the supply portion and the collection portion is formed.

7. The analysis device according to claim 6, wherein the intra-substrate flow path has:
a surface opening provided in the surface of the substrate;
a back surface opening provided in a back surface of the substrate;
a first inner wall provided between the surface opening and the back surface opening and inclined with respect to the back surface of the substrate; and
a second inner wall provided downstream of the first inner wall between the surface opening and the back surface opening and perpendicular to the back surface of the substrate.

8. The analysis device according to any one of claims 4 to 7, wherein
the inflow opening portion has a smallest opening area in the flow channel, and
the sample is analyzed by a change in current value at a time when the sample passes through the inflow opening portion being detected.

9. The analysis device according to any one of claims 4 to 8, wherein the supply portion and the collection portion are provided in a same surface of the substrate.

10. A fluid chip comprising:
an intra-substrate flow path provided in a substrate;
an insulating film provided on a surface of the substrate; and
an inflow opening portion provided in the insulating film and allowing a sample to flow into the intra-substrate flow path,
wherein the intra-substrate flow path has:
a surface opening provided in the surface of the substrate;
a back surface opening provided in a back surface of the substrate;
a first inner wall provided between the surface opening and the back surface opening and inclined with respect to the back surface of the substrate; and
a second inner wall provided downstream of the first inner wall between the surface opening and the back surface opening and perpendicular to the back surface of the substrate.

11. A fluid chip comprising:
an intra-substrate flow path provided in a substrate;
an insulating film provided on a surface of the substrate;
an inflow opening portion provided in the insulating film and allowing a sample to flow into the intra-substrate flow path; and
a conductive film provided in contact with the insulating film,
wherein the conductive film has a conductive film opening portion connected to the inflow opening portion.
